(19) Europäisches Patentamt / European Patent Office / Office européen des brevets

(11) **EP 3 773 192 B1**

(12) **EUROPÄISCHE PATENTSCHRIFT**

(45) Veröffentlichungstag und Bekanntmachung des Hinweises auf die Patenterteilung:
**18.12.2024 Patentblatt 2024/51**

(21) Anmeldenummer: **19718298.3**

(22) Anmeldetag: **11.04.2019**

(51) Internationale Patentklassifikation (IPC):
*A61B 5/06* (2006.01)  *A61B 5/055* (2006.01)
*A61B 6/03* (2006.01)  *A61B 1/267* (2006.01)
*A61B 5/08* (2006.01)  *A61B 34/20* (2016.01)

(52) Gemeinsame Patentklassifikation (CPC):
**A61B 5/066; A61B 5/489; A61B 5/6852;
A61B 5/7246; A61B 6/032; A61B 6/12;**
A61B 5/055; A61B 5/08; A61B 5/6851; A61B 5/743;
A61B 6/504; A61B 6/5217; A61B 2034/2061;
A61B 2562/0266

(86) Internationale Anmeldenummer:
**PCT/EP2019/059266**

(87) Internationale Veröffentlichungsnummer:
**WO 2019/197534 (17.10.2019 Gazette 2019/42)**

(54) **POSITIONSBESTIMMUNGSVORRICHTUNG ZUM BESTIMMEN EINER POSITION EINES GEGENSTANDS INNERHALB EINER TUBULÄREN STRUKTUR**

POSITION-DETERMINING DEVICE FOR DETERMINING THE POSITION OF AN OBJECT WITHIN A TUBULAR STRUCTURE

DISPOSITIF DE DÉTERMINATION DE POSITION POUR DÉTERMINER LA POSITION D'UN OBJET DANS UNE STRUCTURE TUBULAIRE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **11.04.2018 DE 102018108643**

(43) Veröffentlichungstag der Anmeldung:
**17.02.2021 Patentblatt 2021/07**

(73) Patentinhaber: **Fraunhofer-Gesellschaft zur Förderung
der angewandten Forschung e.V.
80686 München (DE)**

(72) Erfinder:
• **STREHLOW, Jan**
**28359 Bremen (DE)**
• **PÄTZ, Torben**
**28359 Bremen (DE)**
• **HAHN, Horst**
**28359 Bremen (DE)**

(74) Vertreter: **Eisenführ Speiser
Patentanwälte Rechtsanwälte PartGmbB
Postfach 10 60 78
28060 Bremen (DE)**

(56) Entgegenhaltungen:
WO-A1-2018/069462   US-A1- 2014 336 501
US-A1- 2015 254 526   US-A1- 2016 302 869

**Beschreibung**

Die Erfindung betrifft eine Positionsbestimmungsvorrichtung, ein Verfahren und Computerprogramm zum Bestimmen einer Position eines länglichen medizinischen Instruments, wie beispielsweise eines Katheters, Bronchoskops oder Führungsdrahts, innerhalb einer tubulären Struktur, z.B. der Blutgefäße oder Atemwege. Die Erfindung betrifft weiterhin ein Bildgebungssystem, das die Positionsbestimmungsvorrichtung umfasst, sowie ein Bildgebungsverfahren und Bildgebungscomputerprogramm.

In medizinischen Anwendungen sind Positionsbestimmungsvorrichtungen bekannt, die elektromagnetische (EM) oder auf optischer Formmessung ("optical shape sensing", OSS) basierende Trackingtechniken zum Bestimmen einer Position eines interventionellen Instruments innerhalb eines Körpers eines Patienten einsetzen. Die optische Formmessung erfolgt typischerweise mittels Auswertung mehrerer optischer Krümmungssensoren, die wiederum aus konzentrisch um den Querschnitt der Faser angeordneten Dehnungssensoren aufgebaut sind. Die EM- und OSS-Trackingtechniken können jedoch aufgrund von beispielsweise Inhomogenitäten des EM-Felds bzw. Fehlern bei der Interpolation und Integration von Krümmungsinformationen relativ ungenau sein.

Die US-Patentanmeldung US 2015/254526 A1 offenbart ein Registrierungssystem zur Registrierung eines Koordinatensystems eines Formerfassungssystems mit einem Koordinatensystem von pre-prozeduralen oder intra-prozeduralen Bildgebungsdaten. Ein Instrument wie beispielsweise ein Katheter umfasst eine optische Faser mit Formerfassungssensoren, wobei die dreidimensionale Form des Instruments auf Basis von Signalen der Formerfassungssensoren bestimmt wird. Das Koordinatensystem des Formerfassungssystems wird mit dem Koordinatensystem der Bildgebungsdaten registriert, indem eine stabile Krümmung der optischen Faser identifiziert und diese mit einer Krümmung aus den pre-prozeduralen oder intra-prozeduralen Bildgebungsdaten verglichen wird, wobei die verglichenen Krümmungen relative zueinander ausgerichtet werden.

Die US-Patentanmeldung US 2016/0302869 A1 offenbart ein Verfahren, bei dem eine Form eines Instruments bestimmt wird, das sich zumindest teilweise innerhalb eines anatomischen Durchgangs befindet. Ein Satz von Deformationskräften wird für eine Vielzahl von Abschnitten des Instruments bestimmt, wobei dieser Satz von Deformationskräften zusammen mit der bestimmten Form des Instruments und anatomischen Daten, die den anatomischen Durchgang beschreiben, verwendet wird, um ein Kompositmodell zu erzeugen, das eine Position des Instruments relativ zu dem anatomischen Durchgang anzeigt.

Die US-Patentanmeldung US 2014/0336501 A1 offenbart eine diagnostische endoskopische bildgebende Unterstützungsvorrichtung, die Formdaten von tubulärem Gewebe und endoskopische Wegdaten miteinander vergleicht und das Ergebnis dieses Vergleichs zusammen mit dreidimensionalen Bilddaten und den endoskopischen Wegdaten verwendet, um einen Weg eines Endoskops in einem dreidimensionalen stereoskopischen Bild darzustellen.

Es ist eine Aufgabe der vorliegenden Erfindung, eine Positionsbestimmungsvorrichtung, ein Verfahren und Computerprogramm bereitzustellen, die eine verbesserte Bestimmung einer Position eines länglichen medizinischen Instruments innerhalb einer tubulären Struktur ermöglichen. Eine weitere Aufgabe der vorliegenden Erfindung ist es, ein Bildgebungssystem, das die Positionsbestimmungsvorrichtung umfasst, sowie ein Bildgebungsverfahren und ein Bildgebungscomputerprogramm bereitzustellen.

Die Aufgabe wird durch eine Positionsbestimmungsvorrichtung zum Bestimmen einer Position eines länglichen medizinischen Instruments innerhalb einer tubulären Struktur gemäß Anspruch 1 gelöst, wobei die Positionsbestimmungsvorrichtung umfasst:

- eine erste Bereitstellungseinheit zum Bereitstellen einer ersten Verteilung von Krümmungswerten an mehreren ersten Stellen entlang eines Pfades innerhalb der tubulären Struktur,
- eine zweite Bereitstellungseinheit zum Bereitstellen einer zweiten Verteilung von Dehnungswerten oder von Krümmungswerten an mehreren zweiten Stellen entlang des Instruments,
- eine Positionsbestimmungseinheit zum Bestimmen der Position des Instruments relativ zu dem Pfad auf Basis der ersten und zweiten Verteilungen.

Die Krümmungswerte der ersten Verteilung an den mehreren ersten Stellen entlang der tubulären Struktur und die Dehnungswerte oder Krümmungswerte der zweiten Verteilung an den mehreren zweiten Stellen entlang des Instruments sind lokale Werte. Durch die Bestimmung der Position des Instruments innerhalb der tubulären Struktur auf Grundlage dieser lokalen Informationen und nicht auf Grundlage globaler Informationen kann die Genauigkeit der Bestimmung der Position erheblich verbessert werden, da diese Methode weniger empfindlich für Messfehler ist. Beispielsweise kann die Genauigkeit der Bestimmung der Position gegenüber globalen Messfehlern wie Drift, Feldinhomogenitäten et cetera weniger empfindlich und insbesondere überhaupt nicht empfindlich sein. Darüber hinaus kann die Position des Instruments bestimmt werden, ohne dass zum Beispiel eine Rekonstruktion der Form des Instruments auf Grundlage beispielsweise der Krümmungswerte der zweiten Verteilung notwendig ist.

Die erste Bereitstellungseinheit kann eine Speichereinheit sein, in der die Krümmungswerte der ersten Verteilung gespeichert werden, wobei die erste Bereitstellungseinheit geeignet sein kann, die gespeicherten Krümmungswerte der ersten Verteilung bereitzustellen. Die erste Bereitstellungseinheit kann jedoch auch eine Empfangseinheit zum Empfangen der Krümmungswerte der ersten Verteilung von beispielsweise einer ersten Bestimmungseinheit und zum Bereitstellen der empfangenen Krümmungswerte der ersten Verteilung sein. Darüber hinaus kann die erste Bereitstellungseinheit auch selbst die erste Bestimmungseinheit sein. Auch die zweite Bereitstellungseinheit kann eine Speichereinheit oder eine Empfangseinheit sein. Weiterhin kann die zweite Bereitstellungseinheit eine zweite Bestimmungseinheit sein.

Die Krümmungswerte der ersten Verteilung sind bevorzugt vorgegeben. Sie können auf Grundlage eines Bilds der tubulären Struktur wie beispielsweise eines Computertomographie-Bilds, eines Magnetresonanztomographie-Bilds oder eines anderen Bilds bestimmt worden sein. Die Krümmungswerte der ersten Verteilung können auch auf eine andere Weise bestimmt werden. Beispielsweise können in einem vorherigen Verfahren ein oder mehrere Krümmungssensoren innerhalb der tubulären Struktur bewegt werden, so dass sie sich an bekannten ersten Stellen innerhalb der tubulären Struktur befinden, während die Krümmungen der tubulären Struktur mithilfe der Krümmungssensoren gemessen werden. Ein weiteres Beispiel zur Erzeugung der Krümmungswerte ist die Simulation der Lage des medizinischen Instruments innerhalb der aus den Bilddaten extrahierten tubulären Struktur.

Die tubuläre Struktur kann beispielsweise ein Blutgefäßsystem, ein Atmungssystem, oder eine andere verzweigte tubuläre Struktur sein. Die tubuläre Struktur kann auch eine technische tubuläre Struktur sein, wie beispielsweise ein Pipeline-System, ein Tunnelsystem, ein Kabelkanalsystem et cetera. Das medizinische Instrument ist beispielsweise ein Führungsdraht, ein Katheter oder ein Bronchoskop.

In einer bevorzugten Ausführungsform sind die Krümmungswerte der ersten Verteilung und der zweiten Verteilung skalar. Daher kann die Position des Instruments innerhalb der tubulären Struktur ohne Verwendung von richtungsabhängigen Krümmungsinformationen bestimmt werden. In einer Ausführungsform können an einer jeweiligen Stelle zwei lokale Krümmungen in zwei verschiedene Richtungen bestimmt werden, beispielsweise in Quer- und Vertikalachsenrichtung, und diese beiden lokalen Krümmungen können zu einem skalaren Krümmungswert kombiniert werden, der indikativ ist für die lokale Krümmung an der jeweiligen Stelle. Zum Beispiel kann ein skalarer mittlerer Krümmungswert oder ein skalarer Gaußscher Gesamtkrümmungswert bestimmt werden. Die Krümmungswerte können jedoch auch vektorwertig sein. Beispielsweise können die beiden lokalen Krümmungen wie die Krümmung relativ zur Vertikalachse und die Krümmung relativ zur Querachse als die zwei-komponentige Krümmungswerte an der jeweiligen Stelle angesehen werden.

Es ist bevorzugt, dass die erste Bereitstellungseinheit angepasst ist, eine skalare Verteilung von Krümmungswerten entlang eines Pfades innerhalb der tubulären Struktur als die erste Verteilung bereitzustellen. Die zweite Bereitstellungseinheit ist dann bevorzugt angepasst, als die zweite Verteilung eine Verteilung von skalaren Dehnungswerten an den mehreren zweiten Stellen entlang des Instruments bereitzustellen, so dass die Positionsbestimmungseinheit die Position des Instruments relativ zu dem Pfad auf Basis der skalaren Krümmungswerte der ersten Verteilung und der skalaren Dehnungswerte der zweiten Verteilung bestimmt. Da in dieser Situation nur skalare Dehnungswerte benötigt werden, ist ein relativ geringer technischer Aufwand notwendig, um die Position des Instruments innerhalb der tubulären Struktur zu bestimmen.

Es ist weiter bevorzugt, dass die zweite Bereitstellungseinheit angepasst ist, die zweite Verteilung auf Basis von optischen Signalen optischer Dehnungssensoren zu bestimmen, die entlang des Instruments angeordnet sind. Die optischen Dehnungssensoren sind bevorzugt in eine einzelne optische Einkern-Faser integriert. An jeder Stelle entlang des Instruments ist bevorzugt jeweils nur ein Dehnungssensor vorhanden. Dies ermöglicht die Bestimmung der zweiten Verteilung skalarer Dehnungswerte mit einem weiter reduzierten technischen Aufwand im Vergleich zu Krümmungssensoren, der zudem für den Benutzer relativ leicht handhabbar ist.

Wenn in einer anderen Ausführungsform die zweite Verteilung eine Verteilung von Krümmungswerten ist, so können diese beispielsweise mittels OSS-Krümmungssensoren oder Gruppen von EM- oder RFID-Sensoren bestimmt werden. Die relative Position in Bezug zu anderen Sensoren innerhalb der Sensorgruppe ist für diese Gruppe von EM- oder RFID-Sensoren ausreichend, um die lokalen Krümmungen zu berechnen.

Die erste Bereitstellungseinheit ist angepasst, a) ein Unähnlichkeitsmaß auf die erste Verteilung und die zweite Verteilung für verschiedene mögliche Kandidatenpositionen des Instruments relativ zu dem Pfad anzuwenden, wobei das Unähnlichkeitsmaß angepasst ist, für jede Kandidatenposition des Instruments relativ zu dem Pfad einen Unähnlichkeitswert zu liefern, der indikativ ist für eine Unähnlichkeit der beiden Verteilungen in der jeweiligen Kandidatenposition, und b) die Position des Instruments relativ zu dem Pfad auf Basis der für die unterschiedlichen Kandidatenpositionen bestimmten Unähnlichkeitswerte zu bestimmen. In einer Ausführungsform ist die Positionsbestimmungseinheit angepasst, als die Position des Instruments relativ zu dem Pfad die Kandidatenposition zu bestimmen, für die der kleinste Unähnlichkeitswert bestimmt worden ist. Dies kann bereits zu einer relativ genauen Bestimmung der Position des Instruments relativ zu dem Pfad führen.

Des Weiteren ist die Positionsbestimmungsvorrichtung angepasst, für mehrere Pfade innerhalb der tubulären Struktur, die durch Verzweigung der Struktur entstehen, mehrere erste Verteilungen von Krümmungswerten bereitzustellen, wobei die Positionsbestimmungseinheit angepasst ist, die Position des Instruments relativ zu einem dieser Pfade auf Basis der ersten Verteilungen und der zweiten Verteilung zu bestimmen. Die Positionsbestimmungseinheit ist angepasst, a) für jeden Pfad eine Verteilung von Unähnlichkeitswerten für unterschiedliche Kandidatenpositionen des Instruments relativ zu dem jeweiligen Pfad zu bestimmen und b) die Position des Instruments relativ zu einem der Pfade auf Basis der für die unterschiedlichen Pfade und unterschiedlichen Kandidatenpositionen bestimmten Unähnlichkeitswerte zu bestimmen. Dies kann zu einer genauen Bestimmung der Position des Instruments relativ zu einem der Pfade führen, wenn die tubuläre Struktur mehrere mögliche Pfade aufweist, in der sich das Instrument befinden könnte.

Die zweite Bereitstellungseinheit ist angepasst, für unterschiedliche Zeitpunkte mehrere zweite Verteilungen entlang des Instruments bereitzustellen, wobei die mehreren zweiten Verteilungen mehreren Positionen des Instruments relativ zu einem Pfad der tubulären Struktur entsprechen, wobei die Positionsbestimmungseinheit angepasst ist, a) für jeden Pfad und für jeden Zeitpunkt jeweils eine Verteilung von Unähnlichkeitswerten für unterschiedliche Kandidatenpositionen des Instruments relativ zu dem jeweiligen Pfad zu bestimmen und b) die Positionen des Instruments relativ zu einem der Pfade auf Basis der für die unterschiedlichen Pfade, der unterschiedlichen Zeitpunkte und unterschiedlichen Kandidatenpositionen bestimmten Unähnlichkeitswerte zu bestimmen. Die für die unterschiedlichen Zeitpunkte und die unterschiedlichen Kandidatenpositionen bestimmten Unähnlichkeitswerte sind als eine Karte auffassbar, wobei für unterschiedliche Orte, die durch die jeweiligen Zeitpunkte und Kandidatenpositionen festgelegt sind, in der Karte jeweils ein Unähnlichkeitswert eingetragen ist, wobei die Positionsbestimmungseinheit angepasst ist, a) ein Wegmaß bereitzustellen, das für einen Weg durch die jeweilige Karte, der bei einer Kandidatenposition für einen spätesten Zeitpunkt endet und bei einer Kandidatenposition für einen früheren Zeitpunkt beginnt, einen Wegwert liefert, der von den Unähnlichkeitswerten entlang des jeweiligen Weges abhängt, b) für jeden Pfad einen Weg durch die jeweilige Karte mittels des Wegmaßes zu bestimmen, für den ein minimaler Wegwert bestimmt wird, so dass für unterschiedliche Pfade und damit für unterschiedliche Karten jeweils ein optimaler Weg ermittelt wird, und c) die Positionen des Instruments relativ zu einem der Pfade auf Basis der für die unterschiedlichen Pfade ermittelten optimalen Wege unter Berücksichtigung der für diese optimalen Wege bestimmten Wegwerte zu bestimmen. Die Positionsbestimmungseinheit ist bevorzugt angepasst, ein Wegmaß bereitzustellen, das eine Komponente aufweist, die mit zunehmender Summe der Unähnlichkeitswerte entlang des jeweiligen Weges größer wird. In einer Ausführungsform ist die Positionsbestimmungseinheit angepasst, als die Positionen des Instruments relativ zu einem der Pfade die Positionen zu bestimmen, die durch den Weg mit dem niedrigsten Wegwert von allen optimalen Wegen definiert sind. Dies kann zu einer weiter verbesserten Bestimmung der Position des Instruments innerhalb der tubulären Struktur führen.

Es ist aber auch bevorzugt, dass die Positionsbestimmungseinheit angepasst ist, a) für unterschiedliche Zeitpunkte optimale Wege zu ermitteln, so dass für unterschiedliche Zeitpunkte und unterschiedliche Pfade jeweils ein optimaler Weg mit einem entsprechenden Wegwert bestimmt wird, wobei, um für einen Pfad und einen bestimmten Zeitpunkt einen optimalen Weg mit einem entsprechenden Wegwert zu bestimmen, die für den bestimmten Zeitpunkt und für frühere Zeitpunkte und für die Kandidatenpositionen ermittelten Unähnlichkeitswerte, die als eine Karte für den jeweiligen Pfad und den bestimmten Zeitpunkt auffassbar sind, verwendet werden, b) zu jedem Zeitpunkt von den ermittelten optimalen Wegen den optimalen Weg auszuwählen, für den ein minimaler Wegwert bestimmt worden ist, wobei, bevor der optimale Weg mit dem minimalen Wegwert ausgewählt wird, für einen optimalen Weg, für den zu früheren Zeitpunkten kein minimaler Wegwert im Vergleich zu den Wegwerten, die für die anderen optimalen Wege bestimmt worden sind, bestimmt worden ist, der Wegwert erhöht wird, und c) als die Positionen des Instruments relativ zu einem der Pfade die Positionen zu bestimmen, die durch den ausgewählten optimalen Weg definiert sind. Dies kann zu einer weiter verbesserten Genauigkeit der Bestimmung der Position des Instruments innerhalb der tubulären Struktur führen.

Die Positionsbestimmungseinheit kann angepasst sein, das Unähnlichkeitsmaß anzuwenden, indem a) eine räumliche erste Gradientenverteilung der ersten Verteilung bestimmt wird, b) eine räumliche zweite Gradientenverteilung der zweiten Verteilung bestimmt wird, c) für jeden Ort entlang des Instruments der jeweilige Gradient der zweiten Verteilung mit dem jeweiligen Krümmungsgradienten der ersten Verteilung verglichen wird, wobei die jeweilige Kandidatenposition definiert, welcher jeweilige Gradient der zweiten Verteilung mit welchem jeweiligen Krümmungsgradienten der ersten Verteilung an dem jeweiligen Ort verglichen wird, wobei für den jeweiligen Ort und für die jeweilige Kandidatenposition ein Unter-Unähnlichkeitsmaß verwendet wird, das abhängig ist von i) den Richtungen der Gradienten relativ zueinander und/oder ii) dem Betrag des Gradienten der ersten Verteilung und dem Betrag des Gradienten der zweiten Verteilung, wobei durch die Anwendung des Unter-Unähnlichkeitsmaßes für jeden Ort ein Unter-Unähnlichkeitswert bestimmt wird, und d) die Unter-Unähnlichkeitswerte, die für eine Kandidatenposition bestimmt worden sind, summiert werden, um für die jeweilige Kandidatenposition einen jeweiligen Unähnlichkeitswert zu bestimmen. In einer Ausführungsform nimmt der Wert des Unter-Unähnlichkeitsmaßes mit zunehmender Ähnlichkeit der Richtungen der beiden Gradienten relativ zueinander ab. Diese Abnahme kann monoton und/oder stetig sein.

Zudem liefert in einer Ausführungsform das Unter-Unähnlichkeitsmaße einen ersten Wert, wenn der Betrag des Gradienten der ersten Verteilung kleiner ist als ein erster Betragsschwellwert und der Betrag des Gradienten der zweiten Verteilung kleiner ist als ein zweiter Betragsschwellwert, und einen zweiten Wert, wenn der Betrag des Gradienten der ersten Verteilung nicht kleiner ist als der erste Betragsschwellwert und/oder der Betrag des Gradienten der zweiten Verteilung nicht kleiner ist als der zweite Betragsschwellwert, wobei der erste Wert kleiner ist als der zweite Wert. Das Unter-Unähnlichkeitsmaß kann auch beide Komponente aufweisen, das heißt, eine erste Komponente, die einen Wert liefert, der mit zunehmender Ähnlichkeit der Gradienten abnimmt, und eine zweite Komponente, die einen Wert liefert, der kleiner ist, wenn der Betrag des Gradienten der ersten Verteilung kleiner ist als der erste Betragsschwellwert und der Betrag des Gradienten der zweiten Verteilung kleiner ist als der zweite Betragsschwellwert, und ansonsten einen größeren Wert liefert. Die erste Komponente und die zweite Komponente können beispielsweise linear kombiniert werden, was auch eine einfache Addition einschließt. Diese beiden Komponenten können auch nicht-linear kombiniert werden. In einer Ausführungsform werden die beiden Komponenten multiplikativ kombiniert. Das Ergebnis dieser Kombination ist ein skalarer Wert für das Unter-Unähnlichkeitsmaß.

In einer weiteren Ausführungsform nimmt der Wert des Unter-Unähnlichkeitsmaßes mit abnehmendem Betrag der Gradienten der ersten und zweiten Verteilung ab. Das heißt, dass dieser Wert abnimmt, wenn beide Beträge von beiden Gradienten abnehmen. Diese Abnahme kann monoton und/oder stetig sein. Dieser Wert kann auch mit dem Wert der zuvor genannten ersten Komponente linear oder nicht-linear kombiniert werden. Beispielsweise kann dieser Wert mit dem Wert der oben genannten ersten Komponente multipliziert werden oder diese Werte können addiert werden.

Die Positionsbestimmungseinheit kann aber auch angepasst sein, das Unähnlichkeitsmaß anzuwenden, indem a) lokale Maxima der ersten Verteilung bestimmt werden, b) lokale Maxima der zweiten Verteilung bestimmt werden, c) jedem lokalen Maximum der ersten Verteilung ein lokales Maximum der zweiten Verteilung zugeordnet wird, so dass die Summe aller räumlichen Abstände zwischen zugeordneten lokalen Maxima minimal ist, wobei jedes lokale Maximum der zweiten Verteilung nur einem lokalen Maximum der ersten Verteilung zugeordnet wird, und d) die räumlichen Abstände zwischen den zugeordneten lokalen Maxima der ersten Verteilung und den zugeordneten lokalen Maxima der zweiten Verteilung addiert werden. Ferner kann die Positionsbestimmungseinheit angepasst sein, das Unähnlichkeitsmaß anzuwenden, indem a) für jeden Ort entlang des Instruments der jeweilige Wert der zweiten Verteilung mit dem jeweiligen Wert der ersten Verteilung verglichen wird, wobei die jeweilige Kandidatenposition definiert, welcher jeweilige Wert der zweiten Verteilung mit welchem jeweiligen Wert der ersten Verteilung an dem jeweiligen Ort verglichen wird, wobei für den jeweiligen Ort und für die jeweilige Kandidatenposition ein Unter-Unähnlichkeitsmaß verwendet wird, das Null ist, wenn i) der jeweilige Wert der ersten Verteilung oberhalb eines vorgegebenen ersten Schwellwerts liegt oder ii) der jeweilige Wert der ersten Verteilung unterhalb des ersten Schwellwerts liegt und der jeweilige Wert der zweiten Verteilung unterhalb eines vorgegebenen zweiten Schwellwerts liegt, und das ansonsten einen positiven Wert aufweist, und b) die Unter-Unähnlichkeitswerte, die für eine Kandidatenposition bestimmt worden sind, summiert werden, um für die jeweilige Kandidatenposition einen jeweiligen Unähnlichkeitswert zu bestimmen. Es ist gefunden worden, dass bei Verwendung dieser Unähnlichkeitsmaße die Bestimmung der Position des Instruments in der tubulären Struktur weiter verbessert werden kann. Es ist aber natürlich auch möglich, andere Unähnlichkeitsmaße zu verwenden.

Die Positionsbestimmungseinheit kann auch angepasst sein, das Unähnlichkeitsmaß anzuwenden, indem für jeweilige Kandidatenpositionen eine Kreuzkorrelation, insbesondere eine normalisierte Kreuzkorrelation, auf die erste Verteilung und die zweite Verteilung angewendet wird. Insbesondere ist die Positionsbestimmungseinheit angepasst, den Mittelwert $E_D$ der Dehnungswerte oder Krümmungswerte der zweiten Verteilung, den Mittelwert $E_T$ der Krümmungswerte der ersten Verteilung, die Standardabweichung $\sigma_D$ der Dehnungswerte oder Krümmungswerte der zweiten Verteilung, die Standardabweichung $\sigma_T$ der Krümmungswerte der ersten Verteilung und schließlich die normalisierte Kreuzkorrelation gemäß folgender Formel zu berechnen:

$$\frac{1}{n}\sum_i \frac{1}{\sigma_D \sigma_T}(D(i) - E_D)(T(i) - E_T) \qquad ,$$

wobei n die Anzahl der Krümmungswerte der ersten Verteilung und die Anzahl der Dehnungswerte bzw. Krümmungswerte der zweiten Verteilung ist, wobei der Index $i$ die einzelnen Krümmungswerte der ersten Verteilung und die einzelnen Dehnungswerte bzw. Krümmungswerte der zweiten Verteilung indiziert, wobei $D(i)$ den jeweiligen Dehnungswert bzw. Krümmungswert der zweiten Verteilung und $T(i)$ den jeweiligen Krümmungswert der ersten Verteilung bezeichnet. Der Dehnungswert bzw. Krümmungswert $D(i)$ der zweiten Verteilung und der Krümmungswert $T(i)$ der ersten Verteilung sind jeweils die $i$-ten Werte, die bei der jeweiligen Kandidatenposition übereinanderliegen. Da für unterschiedliche Kandidatenpositionen die erste Verteilung und die zweite Verteilung relativ zueinander unterschiedlich verschoben sind, liegen für unterschiedliche Kandidatenpositionen unterschiedliche erste Stellen der ersten Verteilung und unterschiedliche zweite Stellen der zweiten Verteilung übereinander, womit auch in der Regel unterschiedliche Dehnungs- bzw. Krümmungswerte der zweiten Verteilung und Krümmungswerte der ersten Verteilung übereinanderliegen und sich demnach für unterschiedliche Kandidatenpositionen unterschiedliche normalisierte Kreuzkorrelationen ergeben. Es ist gefunden worden, dass bei Verwendung dieses auf einer normalisierten Kreuzkorrelation basierenden Unähnlichkeitsmaßes die Bestimmung der Position des Instruments in der tubulären Strukturweiter verbessert werden kann.

Die oben genannte Aufgabe wird zudem durch ein Bildgebungssystem gelöst, wobei das Bildgebungssystem aufweist:

- eine Positionsbestimmungsvorrichtung zum Bestimmen einer Position eines länglichen medizinischen Instruments innerhalb einer tubulären Struktur nach Anspruch 1,
- eine bildgebende Einheit zum Bereitstellen eines Bilds der tubulären Struktur,
- eine Visualisierungserzeugungseinheit zum Erzeugen einer Visualisierung der tubulären Struktur auf Grundlage des bereitgestellten Bilds und der bestimmten Position.

Zudem wird die oben genannte Aufgabe durch ein Positionsbestimmungsverfahren zum Bestimmen einer Position eines länglichen medizinischen Instruments innerhalb einer tubulären Struktur nach Anspruch 11 gelöst, wobei das Verfahren umfasst:

- Bereitstellen einer ersten Verteilung von Krümmungswerten an mehreren ersten Stellen entlang eines Pfades innerhalb der tubulären Struktur durch eine erste Bereitstellungseinheit, wobei für mehrere Pfade innerhalb der tubulären Struktur mehrere erste Verteilungen von Krümmungswerten bereitgestellt werden,- Bereitstellen einer zweiten Verteilung von Dehnungswerten oder von Krümmungswerten an mehreren zweiten Stellen entlang des Instruments durch eine zweite Bereitstellungseinheit, wobei für unterschiedliche Zeitpunkte mehrere zweite Verteilungen entlang des Instruments bereitgestellt werden, wobei die mehreren zweiten Verteilungen mehreren Positionen des Instruments relativ zu einem Pfad der tubulären Struktur entsprechen,
- Bestimmen, für jeden Pfad und für jeden Zeitpunkt, von jeweils einer Verteilung von Unähnlichkeitswerten für unterschiedliche Kandidatenpositionen des Instruments relativ zu dem jeweiligen Pfad durch eine Positionsbestimmungseinheit, wobei ein Unähnlichkeitsmaß auf die entsprechende erste Verteilung und die entsprechende zweite Verteilung für verschiedene mögliche Kandidatenpositionen des Instruments relativ zu dem Pfad angewendet wird, wobei das Unähnlichkeitsmaß angepasst ist, für jede Kandidatenposition des Instruments relativ zu dem jeweiligen Pfad einen Unähnlichkeitswert zu liefern, der indikativ ist für eine Unähnlichkeit der beiden Verteilungen in der jeweiligen Kandidatenposition, wobei die für die unterschiedlichen Zeitpunkte und die unterschiedlichen Kandidatenpositionen bestimmten Unähnlichkeitswerte als eine Karte auffassbar sind, wobei für unterschiedliche Orte, die durch die jeweiligen Zeitpunkte und Kandidatenpositionen festgelegt sind, in der Karte jeweils ein Unähnlichkeitswert eingetragen ist,
- Bereitstellen eines Wegmaßes, das für einen Weg durch die jeweilige Karte, der bei einer Kandidatenposition für einen spätesten Zeitpunkt endet und bei einer Kandidatenposition für einen früheren Zeitpunkt beginnt, einen Wegwert liefert, der von den Unähnlichkeitswerten entlang des jeweiligen Weges abhängt, durch die Positionsbestimmungseinheit,
- Bestimmen eines Weges für jeden Pfad durch die jeweilige Karte mittels des Wegmaßes, für den ein minimaler Wegwert bestimmt wird, so dass für unterschiedliche Pfade und damit für unterschiedliche Karten jeweils ein optimaler Weg ermittelt wird, durch die Positionsbestimmungseinheit, und
- Bestimmen der Positionen des Instruments relativ zu einem der Pfade auf Basis der für die unterschiedlichen Pfade ermittelten optimalen Wege unter Berücksichtigung der für diese optimalen Wege bestimmten Wegwerte durch die Positionsbestimmungseinheit.

Weiterhin wird die oben genannte Aufgabe durch ein Bildgebungsverfahren gelöst, wobei das Bildgebungsverfahren umfasst:

- Bereitstellen eines Bilds einer tubulären Struktur durch eine bildgebende Einheit,
- Bestimmen einer Position eines länglichen medizinischen Instruments innerhalb der tubulären Struktur gemäß dem Positionbestimmungsverfahren nach Anspruch 11 durch eine Positionsbestimmungsvorrichtung nach Anspruch 1, und
- Erzeugen einer Visualisierung der tubulären Struktur auf Grundlage des bereitgestellten Bilds und der bestimmten Position durch eine Visualisierungserzeugungseinheit.

Des Weiteren wird die oben genannte Aufgabe durch ein Computerprogramm zum Bestimmen einer Position eines länglichen medizinischen Instruments gelöst, wobei das Computerprogramm Programmcodemittel aufweist, die angepasst sind, die Positionsbestimmungsvorrichtung nach Anspruch 1 dazu zu veranlassen, das Positionsbestimmungsverfahren gemäß Anspruch 11 durchzuführen, wenn es auf der Positionsbestimmungsvorrichtung ausgeführt wird.

Die Programmcodemittel können ausgebildet sein, ein Bildgebungssystem nach Anspruch 10, das die Positionsbestimmungsvorrichtung umfasst, zu veranlassen, folgende Schritte auszuführen, wenn das Computerprogramm auf dem Bildgebungssystem ausgeführt wird: a) Bereitstellen eines Bilds der tubulären Struktur und b) Erzeugen einer Visualisierung der tubulären Struktur auf Grundlage des bereitgestellten Bilds und der bestimmten Position. Mit anderen Worten, die oben genannte Aufgabe kann des Weiteren durch ein Bildgebungscomputerprogramm gelöst werden, das Programmcodemittel zum Veranlassen eines Bildgebungssystems nach Anspruch 10, das Bildgebungsverfahren nach Anspruch 12 auszuführen, wenn das Computerprogramm auf dem Bildgebungssystem ausgeführt wird, umfasst.

Es sollte verstanden werden, dass die Positionsbestimmungsvorrichtung nach Anspruch 1, das Bildgebungssystem nach Anspruch 10, das Positionsbestimmungsverfahren nach Anspruch 11, das Bildgebungsverfahren nach Anspruch 12, das Computerprogramm nach Anspruch 13 und das Bildgebungscomputerprogramm nach Anspruch 14 ähnliche Ausführungsformen aufweisen.

Im Folgenden werden Ausführungsformen unter Bezugnahme auf folgende Figuren beschrieben, wobei

Fig. 1    schematisch und beispielhaft eine Ausführungsform eines Bildgebungssystems zeigt,

Fig. 2    ein Flussdiagramm zeigt, das beispielhaft eine Ausführungsform eines Bildgebungsverfahrens darstellt,

Fig. 3    beispielhaft ein Bild einer tubulären Struktur zeigt,

Fig. 4    beispielhaft einen Gefäßgraphen, der die tubuläre Struktur repräsentiert und aus dem in Fig. 3 gezeigten Bild extrahiert worden ist,

Fig. 5    beispielhaft unterschiedliche erste Verteilungen von Krümmungswerten für unterschiedliche Pfade innerhalb der tubulären Struktur darstellt,

Fig. 6    beispielhaft eine Verteilung von Unähnlichkeitswerten für unterschiedliche Kandidatenpositionen und einen Pfad darstellt,

Fig. 7    beispielhaft Unähnlichkeitswerte für unterschiedliche Kandidatenpositionen und Zeiten für einen Pfad innerhalb der tubulären Struktur darstellt, wobei hierdurch eine Karte gebildet wird,

Fig. 8    beispielhaft einen optimalen Weg durch die in Fig. 7 gezeigte Karte darstellt, und

Fig. 9    beispielhaft verschiedene Kostenkurven zeigt.

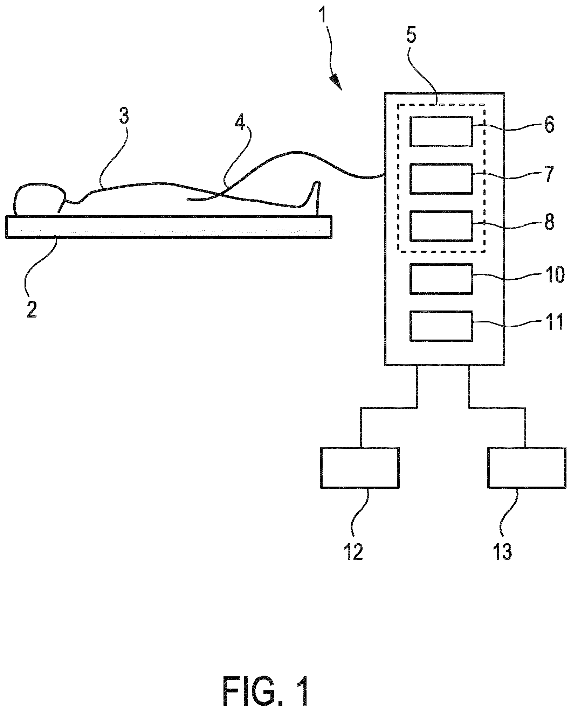

Fig. 1 zeigt schematisch und beispielhaft eine Ausführungsform eines Bildgebungssystems 1, das eine Positionsbestimmungsvorrichtung 5 zum Bestimmen einer Position eines Instruments 4 innerhalb einer tubulären Struktur, eine bildgebende Einheit 10 zum Bereitstellen eines Bilds der tubulären Struktur und eine Visualisierungserzeugungseinheit 11 zum Erzeugen einer Visualisierung der tubulären Struktur auf Grundlage des bereitgestellten Bilds und der bestimmten Position umfasst. In dieser Ausführungsform handelt es sich bei der tubulären Struktur um eine Blutgefäßstruktur eines Patienten 3, der auf einer Patientenliege 2 liegt. Die Positionsbestimmungsvorrichtung 5 umfasst eine erste Bereitstellungseinheit 6 zum Bereitstellen von Krümmungswerten, die indikativ sind für Krümmungen der tubulären Struktur an mehreren ersten Stellen entlang der tubulären Struktur, wobei die erste Bereitstellungseinheit 6 in dieser Ausführungsform geeignet ist, die Krümmungswerte auf Grundlage des von der bildgebenden Einheit 10 bereitgestellten Bilds zu bestimmen. Insbesondere ist die bildgebende Einheit 10 geeignet, ein dreidimensionales Bild der tubulären Struktur wie z. B. ein Computertomographie-Bild oder ein Magnetresonanz-Bild bereitzustellen, welches vor der Einführung des Instruments 4 in den Patienten 3 aufgenommen worden sein kann, d. h. das bereitgestellte Bild kann ein prä-interventionelles dreidimensionales Bild sein. Die erste Bereitstellungseinheit 6 kann geeignet sein, die tubuläre Struktur innerhalb des bereitgestellten Bilds zu segmentierten und die Krümmungswerte auf Grundlage der Segmentierung der tubulären Struktur in dem Bild zu bestimmen. Diese Krümmungswerte sind lokale Krümmungswerte und bilden eine erste Verteilung.

In dieser Ausführungsform ist das Instrument 4 ein Katheter, der an mehreren zweiten Stellen entlang des Katheters 4 optische Dehnungssensoren aufweist. Die Positionsbestimmungsvorrichtung 5 umfasst eine zweite Bereitstellungseinheit 7 zum Bereitstellen von Dehnungswerten auf Grundlage von optischen Signalen, die von den Dehnungssensoren empfangen wurden. Die Dehnungswerte sind skalar und indikativ für die Dehnungen an den jeweiligen zweiten Stellen und sind daher lokale Dehnungswerte.

Die Positionsbestimmungsvorrichtung 5 umfasst weiterhin eine Positionsbestimmungseinheit 8 zum Bestimmen der Position des Instruments 4 innerhalb der tubulären Struktur auf Grundlage der ersten Krümmungswerte und der zweiten Dehnungswerte. In dieser Ausführungsform sind diese ersten und zweiten Werte skalar. Insbesondere können an einer bestimmten räumlichen Stelle zwei lokale Krümmungen der tubulären Struktur in zwei verschiedenen Richtungen bestimmt werden und diese beiden lokalen Krümmungen können zu einem skalaren Krümmungswert kombiniert werden, der indikativ ist für die lokale Krümmung an der jeweiligen räumlichen Stelle. Beispielsweise kann gemäß der folgenden Gleichung ein skalarer mittlerer Krümmungswert bestimmt werden:

$$K_m = \frac{1}{2} \cdot (k_1 + k_2)$$

$$,\qquad\qquad (1)$$

wobei $K_m$ der skalare mittlere Krümmungswert ist und $k_1$ $k_2$ die lokalen Krümmungen in den beiden unterschiedlichen Richtungen sind. Außerdem kann ein (Gaußscher) Krümmungswert $K_t$ gemäß der folgenden Gleichung berechnet werden:

$$K_t = k_1 k_2$$

$$.\qquad\qquad (2)$$

Die Variablen $k_1$ und $k_2$ können als die Hauptkrümmungen beispielsweise der tubulären Struktur angesehen werden, d. h. die Eigenwerte des Formoperators dertubulären Struktur an der jeweiligen Stelle.

Die Positionsbestimmungseinheit 8 ist angepasst, ein Unähnlichkeitsmaß auf die erste Verteilung und die zweite Verteilung für verschiedene mögliche Kandidatenpositionen des Katheters 4 relativ zu dem Pfad anzuwenden, wobei das Unähnlichkeitsmaß angepasst ist, für jede Kandidatenposition des Katheters 4 relativ zu dem Pfad einen Unähnlichkeitswert zu liefern, der indikativ ist für eine Unähnlichkeit der beiden Verteilungen in den jeweiligen Kandidatenpositionen. Die Positionsbestimmungseinheit 8 ist zudem angepasst, die Position des Katheters 4 relativ zu dem Pfad auf Basis der für die unterschiedlichen Kandidatenpositionen bestimmten Unähnlichkeitswerte zu bestimmen. Die Positionsbestimmungseinheit 8 kann beispielsweise angepasst sein, als die Position des Katheters 4 relativ zu dem Pfad die Kandidatenposition zu bestimmen, für die der kleinste Unähnlichkeitswert bestimmt worden ist.

Die Positionsbestimmungsvorrichtung ist zudem angepasst, auch dann die Position des Katheters 4 in der tubulären Struktur zu bestimmen, wenn die tubuläre Struktur mehrere Pfade aufweist. Insbesondere ist die erste Bereitstellungseinheit 6 angepasst, für mehrere Pfade innerhalb der tubulären Struktur mehrere erste Verteilungen von Krümmungswerten bereitzustellen, wobei die Positionsbestimmungseinheit 8 angepasst ist, die Position des Katheters 4 relativ zu einem dieser Pfade auf Basis der ersten Verteilungen und auf Basis der zweiten Verteilung zu bestimmen. Bevorzugt ist die Positionsbestimmungseinheit 8 angepasst, für jeden Pfad eine Verteilung von Unähnlichkeitswerten für unterschiedliche Kandidatenpositionen des Katheters 4 relativ zu dem jeweiligen Pfad zu bestimmen und die Position des Katheters 4 relativ zu einem der Pfade auf Basis der für die unterschiedlichen Pfade und unterschiedlichen Kandidatenpositionen bestimmten Unähnlichkeitswerte zu bestimmen.

Zudem ist die zweite Bereitstellungseinheit 7 angepasst, für unterschiedliche Zeitpunkte mehrere zweite Verteilungen entlang des Katheters 4 bereitzustellen, wobei die mehreren zweiten Verteilungen mehreren Positionen des Katheters 4 relativ zu einem Pfad der tubulären Struktur entsprechen. Die Positionsbestimmungseinheit 8 ist angepasst, für jeden Pfad und für jeden Zeitpunkt jeweils eine Verteilung von Unähnlichkeitswerten für unterschiedliche Kandidatenpositionen des medizinischen Instruments relativ zu dem jeweiligen Pfad zu bestimmen und die Positionen des Katheters 4 relativ zu einem der Pfade auf Basis der für die unterschiedlichen Pfade, die unterschiedlichen Zeitpunkte und unterschiedlichen Kandidatenpositionen bestimmten Unähnlichkeitswerte zu bestimmen. Insbesondere sind die für die unterschiedlichen Zeitpunkte und die unterschiedlichen Kandidatenpositionen bestimmten Unähnlichkeitswerte als eine Karte auffassbar, wobei für unterschiedliche Orte, die durch die jeweiligen Zeitpunkte und Kandidatenpositionen festgelegt sind, in der Karte jeweils ein Unähnlichkeitswert eingetragen ist. Die Positionsbestimmungseinheit 8 ist dann angepasst, ein Wegmaß bereitzustellen, das für einen Weg durch die jeweilige Karte, der bei einer Kandidatenposition für einen spätesten Zeitpunkt endet und bei einer Kandidatenposition für einen früheren Zeitpunkt beginnt, einen Wegwert liefert, der von den Unähnlichkeitswerten entlang des jeweiligen Weges abhängt. Die Positionsbestimmungseinheit 8 ist zudem angepasst, für jeden Pfad einen Weg durch die jeweilige Karte mittels des Wegmaßes zu bestimmen, für den ein minimaler Wegwert bestimmt wird, so dass für unterschiedliche Pfade jeweils ein optimaler Weg ermittelt wird. Außerdem ist die Positionsbestimmungseinheit 8 angepasst, die Positionen des Katheters 4 relativ zu einem der Pfade auf Basis der für die unterschiedlichen Pfade ermittelten optimalen Wege unter Berücksichtigung der für diese optimalen Wege bestimmten Wegwerte zu bestimmen.

Die Positionsbestimmungseinheit 8 ist insbesondere angepasst, ein Wegmaß bereitzustellen, das mit zunehmender Summe der Unähnlichkeitswerte entlang des jeweiligen Weges größer wird. Zudem ist die Positionsbestimmungseinheit 8 bevorzugt angepasst, als Position des Katheters 4 relativ zu einem der Pfade die Positionen zu bestimmen, die durch den Weg mit dem niedrigsten Wegwert von allen optimalen Wegen definiert sind.

Die Positionsbestimmungseinheit 8 ist zudem bevorzugt angepasst, für unterschiedliche Zeitpunkte optimale Wege zu ermitteln, so dass für unterschiedliche Zeitpunkte und für unterschiedliche Pfade jeweils ein optimaler Weg mit einem entsprechenden Wegwert bestimmt wird, wobei, um für einen Pfad und einen bestimmten Zeitpunkt einen optimalen Weg mit einem entsprechenden Wegwert zu bestimmen, die für den bestimmten Zeitpunkt und für frühere Zeitpunkte und für die Kandidatenpositionen ermittelten Unähnlichkeitswerte, die als eine Karte für den jeweiligen Pfad und den bestimmten Zeitpunkt auffassbar sind, verwendet werden. Zudem ist die Positionsbestimmungseinheit 8 bevorzugt angepasst, zu jedem Zeitpunkt von den ermittelten optimalen Wegen den optimalen Weg auszuwählen, für den ein minimaler Wegwert bestimmt worden ist, wobei, bevor der optimale Weg mit dem minimalen Wegwert ausgewählt wird, für einen optimalen Weg, für den zu früheren Zeitpunkten kein minimaler Wegwert im Vergleich zu den Wegwerten, die für die anderen optimalen Wege bestimmt worden sind, bestimmt worden ist, der Wegwert erhöht wird. Zudem ist die Positionsbestimmungseinheit 8 bevorzugt angepasst, als die Positionen des Katheters 4 relativ zu einem der Pfade die Positionen zu bestimmen, die durch den ausgewählten optimalen Weg definiert sind.

Zum Bestimmen eines Unähnlichkeitswertes kann die Positionsbestimmungseinheit 8 angepasst sein, Gradienten des gemessenen Dehnungssensorprofils und Gradienten des Krümmungsprofils der tubulären Struktur zu berechnen. Die Positionsbestimmungseinheit 8 kann demnach angepasst sein, das Unähnlichkeitsmaß anzuwenden, indem eine räumliche erste Gradientenverteilung der ersten Verteilung und eine räumliche zweite Gradientenverteilung der zweiten Verteilung bestimmt werden. Die Positionsbestimmungseinheit 8 ist dann zudem angepasst, für jeden Ort entlang des Katheters 4 den jeweiligen Gradienten der zweiten Verteilung mit dem jeweiligen Krümmungsgradienten der ersten Verteilung zu vergleichen, wobei die jeweilige Kandidatenposition definiert, welcher jeweilige Gradient der zweiten Verteilung mit welchem jeweiligen Krümmungsgradienten der ersten Verteilung an dem jeweiligen Ort verglichen wird und wobei für den jeweiligen Ort und für die jeweilige Kandidatenposition ein Unter-Unähnlichkeitsmaß verwendet wird, das mit zunehmender Ähnlichkeit der Richtungen der Gradienten relativ zueinander abnimmt. Diese Abnahme mit zunehmender Ähnlichkeit kann beispielsweise monoton sein. Alternativ oder zusätzlich kann das Unter-Unähnlichkeitsmaß einen kleineren Wert liefern, wenn der Betrag des Gradienten der ersten Verteilung kleiner ist als ein vorgegebener erster Betragsschwellwert und der Betrag des Gradienten der zweiten Verteilung kleiner ist als ein vorgegebener zweiter Betragsschwellwert, und ansonsten einen größeren Wert liefern. Das Unter-Unähnlichkeitsmaß kann demnach zumindest eine oder zumindest zwei Komponenten aufweisen, wobei eine erste Komponente einen Wert liefert, der mit zunehmender Ähnlichkeit der Richtungen der Gradienten abnimmt, und/oder wobei eine zweite Komponente einen Wert liefert, der kleiner ist, wenn der Betrag des Gradienten der ersten Verteilung kleiner ist als ein vorgegebener erster Betragsschwellwert und der Betrag des Gradienten der zweiten Verteilung kleiner ist als ein vorgegebener zweiter Betragsschwellwert, und der ansonsten einen größeren Wert liefert. Das heißt, dass für alle korrespondierenden Gradientenwerte ein Strafterm berechnet wird, der hoch ist, wenn a) beide Gradienten in unähnliche Richtungen zeigen und/oder b) keiner der beiden Gradienten klein ist, wobei mittels eines Vergleichs mit einem entsprechenden Betragsschwellwert bestimmt wird, ob der jeweilige Gradientenwert klein ist.

Durch die Anwendung des Unter-Unähnlichkeitsmaßes wird für jeden Ort ein Unter-Unähnlichkeitswert bestimmt. Die Positionsbestimmungseinheit 8 ist zudem angepasst, die Unter-Unähnlichkeitswerte, die für eine Kandidatenposition bestimmt worden sind, zu summieren, um für die jeweilige Kandidatenposition einen jeweiligen Unähnlichkeitswert zu bestimmen. Das heißt also, dass die Summe aller Strafterme ein Maß ist für die Übereinstimmung von dem Dehnungssensorprofil mit dem Krümmungsprofil.

Die Positionsbestimmungseinheit 8 kann auch angepasst sein, ein anderes Unähnlichkeitsmaß anzuwenden, indem lokale Maxima der ersten Verteilung und lokale Maxima der zweiten Verteilung bestimmt werden. Das heißt, die Positionsbestimmungseinheit 8 kann angepasst sein, alle lokalen Maxima in dem Dehnungssensorprofil und dem Krümmungsprofil zu finden. Die Positionsbestimmungseinheit 8 ist dann ferner angepasst, jedem lokalen Maximum der ersten Verteilung ein lokales Maximum der zweiten Verteilung zuzuordnen, so dass die Summe aller räumlichen Abstände zwischen zugeordneten lokalen Maxima minimal ist, wobei jedes lokale Maximum der zweiten Verteilung nur einem lokalen Maximum der ersten Verteilung zugeordnet wird. Hierzu kann die Positionsbestimmungseinheit 8 angepasst sein, beispielsweise einem Brute-Force-Ansatz zur Zuordnung anzuwenden. Die Positionsbestimmungseinheit 8 ist dann weiter angepasst, die räumlichen Abstände zwischen den zugeordneten lokalen Maxima der ersten Verteilung und den zugeordneten lokalen Maxima der zweiten Verteilung zu addieren, um einen Unähnlichkeitswert zu bestimmen.

Die Positionsbestimmungseinheit 8 kann aber auch angepasst sein, das Unähnlichkeitsmaß anzuwenden, indem für jeden Ort entlang des Katheters 4 der jeweilige Wert der zweiten Verteilung mit dem jeweiligen Wert der ersten Verteilung verglichen wird, wobei die jeweilige Kandidatenposition definiert, welcher jeweilige Wert der zweiten Verteilung mit welchem jeweiligen Wert der ersten Verteilung an dem jeweiligen Ort verglichen wird, wobei für den jeweiligen Ort und für die jeweilige Kandidatenposition ein Unter-Unähnlichkeitsmaß verwendet wird, das Null ist, wenn a) der jeweilige Wert der ersten Verteilung oberhalb eines vordefinierten ersten Schwellwerts liegt oder b) der jeweilige Wert der ersten Verteilung unterhalb des ersten Schwellwerts liegt und der jeweilige Wert der zweiten Verteilung unterhalb eines vordefinierten zweiten Schwellwerts liegt, und das ansonsten einen positiven Wert liefert. Dies basiert auf der Beobachtung, dass Nullstellen der Krümmung in der ersten Verteilung entsprechende Nullstellen in der Dehnungsmessung zur Folge haben. Dieses Unähnlichkeitsmaß kann auch als ein injektiver Nullstellenvergleich betrachtet werden, wobei eine binäre Faltung zwischen kleinen Krümmungen und kleinen Dehnungswerten berechnet wird. Die Unter-Unähnlichkeitswerte, die für eine Kandidatenposition bestimmt worden sind, werden wiederum summiert, um für die jeweilige Kandidatenposition einen jeweiligen Unähnlichkeitswert zu bestimmen.

Die Positionsbestimmungseinheit 8 kann auch angepasst sein, das Unähnlichkeitsmaß anzuwenden, indem für die jeweilige Kandidatenposition eine Kreuzkorrelation, insbesondere eine normalisierte Kreuzkorrelation auf die erste Verteilung und die zweite Verteilung angewendet wird. Insbesondere kann die Positionsbestimmungseinheit 8 angepasst sein, den Mittelwert $E_D$ der Dehnungswerte der zweiten Verteilung, den Mittelwert $E_T$ der Krümmungswerte der ersten Verteilung, die Standardabweichung $\sigma_D$ der genannten Dehnungswerte, die Standardabweichung $\sigma_T$ der genannten Krümmungswerte und schließlich die normalisierte Kreuzkorrelation gemäß folgender Formel zu berechnen:

$$\frac{1}{n}\sum_i \frac{1}{\sigma_D\sigma_T}(D(i) - E_D)(T(i) - E_T) \qquad ,$$

wobei n die Anzahl der Krümmungswerte und damit in diesem Beispiel auch die Anzahl der Dehnungswerte ist, wobei der Index $i$ die einzelnen Krümmungswerte und die einzelnen Dehnungswerte indiziert, wobei $D(i)$ den jeweiligen Dehnungswert und $T(i)$ den jeweiligen Krümmungswert bezeichnet. Der Dehnungswert $D(i)$ und der Krümmungswert $T(i)$ sind jeweils die $i$-ten Werte, die bei der jeweiligen Kandidatenposition übereinanderliegen. Da für unterschiedliche Kandidatenpositionen die erste Verteilung und die zweite Verteilung relativ zueinander unterschiedlich verschoben sind, liegen für unterschiedliche Kandidatenpositionen unterschiedliche erste Stellen der ersten Verteilung und unterschiedliche zweite Stellen der zweiten Verteilung übereinander, womit auch in der Regel unterschiedliche Dehnungswerte und Krümmungswerte übereinanderliegen und sich demnach für unterschiedliche Kandidatenpositionen unterschiedliche normalisierte Kreuzkorrelationen ergeben. In diesem Beispiel ist das Unähnlichkeitsmaß die normalisierte Kreuzkorrelation oder hängt von dieser ab.

Die Visualisierungserzeugungseinheit 11 ist geeignet, die Visualisierung der tubulären Struktur auf Grundlage des bereitgestellten Bilds und der bestimmten Position des Instruments 4 innerhalb der tubulären Struktur so zu erzeugen, dass die bestimmte Position des Instruments 4 in dem bereitgestellten Bild oder in einem weiteren Bild, das aus dem von der bildgebenden Einheit bereitgestellten Bild gewonnen wurde und die gewünschten Strukturen innerhalb des Patienten zeigt, visualisiert wird. Das Bildgebungssystem 1 umfasst weiterhin eine Ausgabeeinheit 13 wie ein Display zur Anzeige der Visualisierung für einen Anwender und eine Eingabeeinheit 12 wie eine Tastatur, eine Computermaus, ein Touchpad usw. Während sich das Instrument 4 daher innerhalb der tubulären Struktur des Patienten 3 befindet, kann der Anwender genau sehen, wo genau sich das Instrument 4 innerhalb der tubulären Struktur befindet, was für den Anwender eine Hilfe bei der Verwendung des Instruments 4 innerhalb des Patienten darstellt. Beispielsweise kann das Instrument 4 über Detektions- und/oder Behandlungseigenschaften verfügen, und die angezeigte Visualisierung kann den Anwender bei der Durchführung von Detektions-und/oder Behandlungsverfahren unterstützen, indem die exakte Position beispielsweise der Spitze des Instruments 4 relativ zu der tubulären Struktur auf der Ausgabeeinheit 13 angezeigt wird.

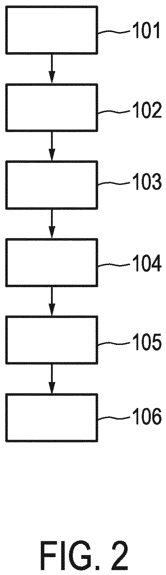

Im Folgenden ist eine Ausführungsform eines Bildgebungsverfahrens beispielhaft mit Bezug auf das in Fig. 2 gezeigte Flussdiagramm beschrieben.

In Schritt 101 wird ein Bild der tubulären Struktur von der bildgebenden Einheit 10 bereitgestellt und in Schritt 102 werden die Krümmungswerte, die indikativ sind für Krümmungen der tubulären Struktur an mehreren ersten Stellen entlang der tubulären Struktur, von der ersten Bereitstellungseinheit 6 bereitgestellt. Die erste Bereitstellungseinheit 6 stellt demnach die erste Verteilung bereit. Diese Krümmungswerte können vor, während oder nach der Einführung des Instruments 4 in die tubuläre Struktur des Patienten 3 bereitgestellt werden. Nachdem das Instrument 4 in die tubuläre Struktur des Patienten 3 eingeführt wurde, werden in Schritt 103 Dehnungswerte, die indikativ sind für Dehnungen des Instruments 4 an mehreren zweiten Stellen entlang des Instruments 4, von der zweiten Bereitstellungseinheit 6 bereitgestellt. Die zweite Bereitstellungseinheit 7 stellt demnach die zweite Verteilung bereit. In Schritt 104 wird die Position des Instruments 4 innerhalb der tubulären Struktur auf Grundlage der ersten und zweiten Verteilungen durch die Positionsbestimmungseinheit 8 bestimmt und in Schritt 105 wird eine Visualisierung der tubulären Struktur auf Grundlage des bereitgestellten Bilds und der bestimmten Position durch die Visualisierungserzeugungseinheit 11 erzeugt, wobei die erzeugte Visualisierung in Schritt 106 auf der Ausgabeeinheit 13 angezeigt wird. Die Schritte 102 bis 104 können als Schritte eines Positionsbestimmungsverfahrens zum Bestimmen einer Position eines Instruments innerhalb einer tubulären Struktur angesehen werden.

Die oben beschriebene Positionsbestimmungsvorrichtung ermöglicht eine endo-tubuläre Navigation auf Basis von Dehnungsprofilen, die beispielsweise mittels faseroptischer Dehnungssensoren bestimmt werden, die in ein medizinisches Instrument wie einen Katheter, einen Führungsdraht, ein Bronchoskop oder ein anderes medizinisches Instrument integriert sein können.

Die beschriebene Positionsbestimmungsvorrichtung und auch das Positionsbestimmungsverfahren sind bevorzugt so angepasst, dass sie eine echtzeitfähige Korrelation der Dehnungsmessung mit einer gegebenen röhrenförmigen Struktur, das heißt der tubulären Struktur, ermöglichen. Die röhrenförmige Struktur wird bevorzugt aus prä-interventionellen Bilddaten extrahiert, wobei basierend auf der extrahierten röhrenförmigen Struktur für jeden möglichen Pfad durch die röhrenförmige Struktur ein skalares Krümmungsprofil des medizinischen Instruments bestimmt wird. Dies kann beispielsweise durch Extraktion der jeweiligen Gefäßmittellinie oder einer biophysikalischen Simulation der zu erwartenden Instrumentenlage im Gefäß erfolgen. Diese bei mehreren Pfaden mehrere Krümmungsprofile werden anschließend als erste Verteilungen dem intra-interventionellen Mappingschritt zur Verfügung gestellt.

Während der Intervention wird das Dehnungsprofil des medizinischen Instruments, das heißt die zweite Verteilung, bestimmt und mit den vorab bestimmten Krümmungsprofilen, d.h. den ersten Verteilungen, korreliert. Dabei werden Abstandsmaße, d.h. Unähnlichkeitsmaße, zwischen dem Dehnungsprofil und dem jeweiligen Krümmungsprofil bevorzugt so gewählt, dass der nichtlineare Zusammenhang zwischen Krümmung und Dehnung berücksichtigt wird.

Wenn als die zweite Verteilung ein Dehnungsprofil entlang des medizinischen Instruments verwendet wird, ist es nicht notwendig, ein Krümmungsprofil auf Basis von mindestens zwei Dehnungsprofilen zu bestimmen, um auf Basis des Krümmungsprofils beispielsweise eine dreidimensionale Form des medizinischen Instruments zu bestimmen, die dann auf die tubuläre Struktur abgebildet werden könnte. Es genügt daher eine einzelne optische Faser bzw. ein einzelner optischer Faserkern mit Dehnungssensoren, um die Position des medizinischen Instruments zu bestimmen, die relativ kostengünstig herstellbar sind und sich auch relativ leicht in ein medizinisches Instrument integrieren lassen.

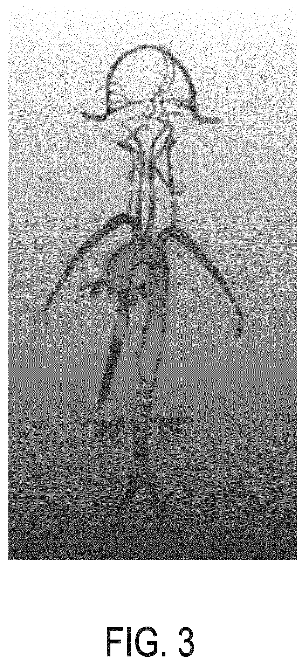

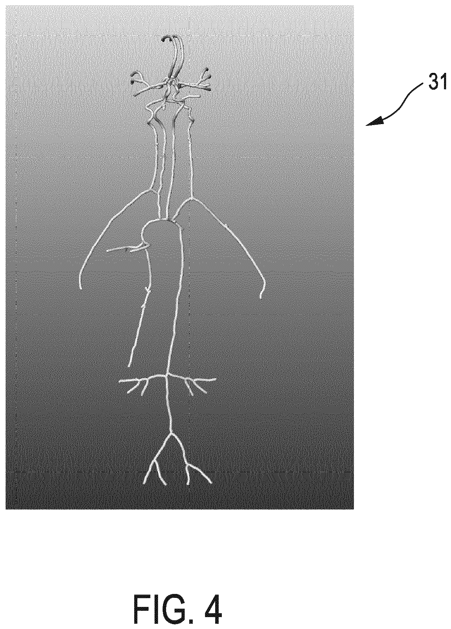

Fig. 3 zeigt beispielhaft ein dreidimensionales Bild einer tubulären Struktur und Fig. 4 illustriert einen Gefäßgraphen 31, der auf Basis des in Fig. 3 gezeigten Bildes erzeugt worden ist und die tubuläre Struktur repräsentiert. Der Gefäßgraph 31 und damit die tubuläre Struktur sind verzweigt und nicht zyklisch.

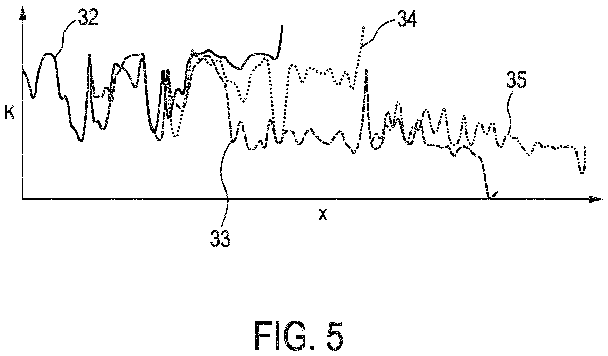

Fig. 5 illustriert beispielhaft vier Krümmungswerteprofile 32...35 für vier Pfade innerhalb der tubulären Struktur, wobei entlang der vertikalen Achse die Krümmung K aufgetragen ist und entlang der horizontalen Achse die jeweilige Stelle x entlang des jeweiligen Pfades. Das heißt, dass für jede Stelle entlang des entsprechenden Pfades eine lokale Krümmung auf Basis der tubulären Struktur berechnet wird, wobei sich aufgrund der Verzweigungen innerhalb der tubulären Struktur die verschiedenen Pfade ergeben, die auch als Gefäßbaumpfade (GBP) bezeichnet werden können. Die lokale Krümmung wird bevorzugt entlang aller GBPs bestimmt. Fig. 5 zeigt demnach mehrere erste Verteilungen.

Die Dehnungssensoren, die entlang des medizinischen Instruments angeordnet sind, werden verwendet, um punktweise Dehnungswerte zu bestimmen, die mit der Krümmung der tubulären Struktur korrelieren können. Hierbei wird angemerkt, dass, je nach Orientierung der optischen Faser relativ zu dem jeweiligen Pfad, einzelne lokale Dehnungssensoren durch die Krümmung des Pfades nicht beeinflusst werden können. Dies ist insbesondere der Fall, wenn die Krümmung orthogonal zur Lageachse des Dehnungssensors im medizinischen Instrument vorhanden ist. Eine genaue Bestimmung der Position des medizinischen Instruments innerhalb der tubulären Struktur ist dennoch mittels der Positionsbestimmungsvorrichtung und dem Positionsbestimmungsverfahren möglich.

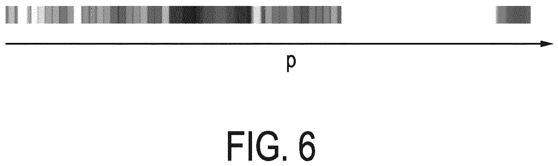

Bevorzugt bestimmt die Positionsbestimmungseinheit 8 in einem ersten Schritt die plausibelste Position des medizinischen Instruments für jeden möglichen GBP. Hierzu werden für jede mögliche Position des medizinischen Instruments, d.h. für jede Kandidatenposition, entlang eines GBPs die Krümmungswerte entlang der jeweiligen ersten Verteilung des jeweiligen GBPs und die gemessenen Dehnungswerte im medizinischen Instrument über ein Distanzmaß, d.h. über ein Unähnlichkeitsmaß, verglichen. Es sind verschiedene Distanzmaße denkbar, wobei bei jedem möglichen Distanzmaß bzw. Unähnlichkeitsmaß Charakteristika aus den Krümmungs- und Dehnungsprofilen, d.h. aus den ersten und zweiten Verteilungen, extrahiert werden und auf Basis dieser Charakteristika eine Distanz, d.h. ein Unähnlichkeitswert, der ersten und zweiten Verteilungen für eine bestimmte Kandidatenposition bestimmt wird. Pro GBP durch die tubuläre Struktur ergibt sich damit eine Plausibilität, d.h. ein Unähnlichkeitswert, jeder möglichen Position, d.h. jeder Kandidatenposition, des medizinischen Instruments entlang des jeweiligen GBPs. Fig. 6 illustriert beispielhaft die Plausibilität bzw. den Unähnlichkeitswert als unterschiedliche Grauwerte für unterschiedliche Kandidatenpositionen p.

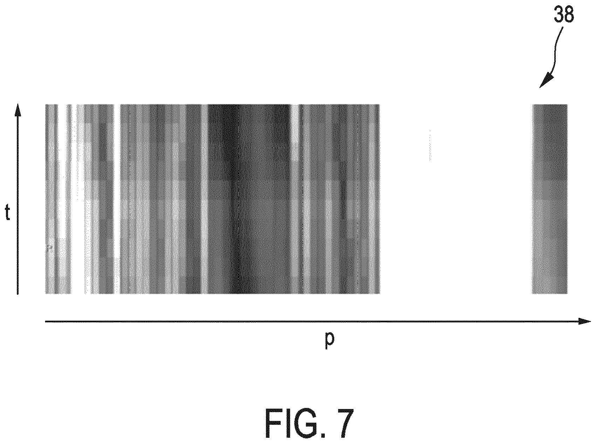

In einer Ausführungsform kann als die plausibelste Kandidatenposition des medizinischen Instruments in einem bestimmten GBP die Kandidatenposition aufgefasst werden, für die der niedrigste Unähnlichkeitswert bestimmt worden ist, d.h. für die die niedrigste Distanz bestimmt worden ist. Aufgrund von beispielsweise Rauschen kann eine derartige Positionsbestimmung allerdings fehlerhaft sein, so dass bevorzugt Wissen über die vergangenen Zeitpunkte mit in die Bestimmung der plausibelsten Kandidatenpositionen integriert wird. Es wird daher bevorzugt für jeden GBP und für jeden Zeitpunkt jeweils eine Verteilung von Unähnlichkeitswerten für unterschiedliche Kandidatenpositionen des medizinischen Instruments relativ zu dem jeweiligen GBP bestimmt, wobei sich hierdurch eine Karte ergibt, die auch als Energiekarte (EKP) bezeichnet werden könnte und die in Fig. 7 illustriert ist.

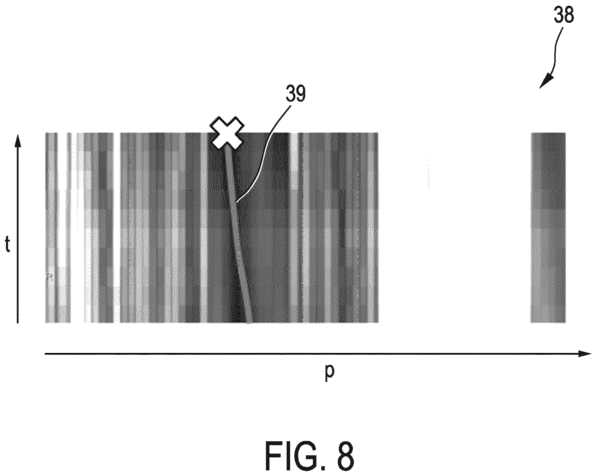

In Fig. 7 sind wiederum die unterschiedlichen Unähnlichkeitswerte der EKP 38 mit unterschiedlichen Grauwerten dargestellt, wobei die EKP 38 durch unterschiedliche Kandidatenpositionen p und unterschiedlichen Zeiten t aufgespannt wird. Das heißt, die EKP 38 umfasst für unterschiedliche Kandidatenpositionen p und unterschiedliche Zeiten t entsprechende Unähnlichkeitswerte, die in Fig. 7 in Graustufen dargestellt sind. Die plausibelste Position des medizinischen Instruments in einem GBP ergibt sich dann bevorzugt durch einen optimalen Weg durch die EKP 38. Optimal bedeutet dabei bevorzugt, dass der akkumulierte Unähnlichkeitswert entlang des Weges durch die EKP 38, optional unter Berücksichtigung einer Regularitätsbedingung, minimal ist. Die optionale Regularitätsbedingung könnte bspw. berücksichtigen, dass der Weg durch die EKP 38 möglichst kurz sein sollte. Es könnte auch berücksichtigt werden, dass der Weg durch die EKP 38 möglichst gerade sein sollte. Technisch ist jeder Weg durch die EKP 38 mit gewissen Kosten assoziiert, die sich aus den akkumulierten Unähnlichkeitswerten und der optionalen Regularität ergeben. Der Weg mit den geringsten Kosten ist der optimale Weg, der die Position des medizinischen Instruments für einen GBP und für unterschiedliche Zeiten definiert. Ein derartiger optimaler Weg 39 ist in Fig. 8 beispielhaft dargestellt.

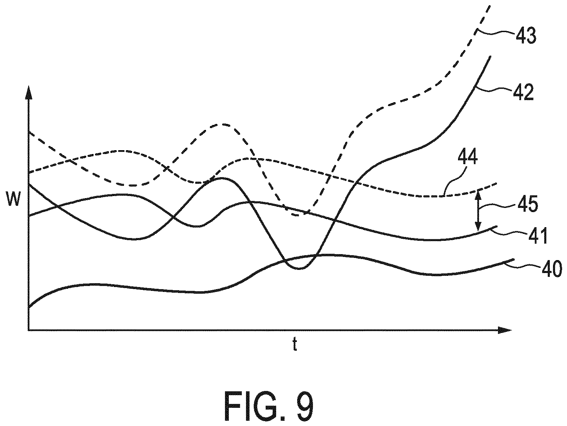

Aus den plausibelsten Positionen pro GBP wird nun die Position des medizinischen Instruments in der gesamten tubulären Struktur bestimmt. Dazu werden die optimalen EKP-Kosten-Werte, d.h. die Wegwerte der optimalen Wege, der einzelnen GBPs miteinander verglichen. In einer Ausführungsform wird der GBP und die dazugehörige plausibelste Position mit dem geringsten Wegwert ausgewählt. Dieser Ansatz ist aber auch rauschbehaftet, so dass dies zu einer fehlerhaften Fehlerbestimmung führen kann, insbesondere da die final bestimmte Position des medizinischen Instruments zwischen GBPs hin und her springen kann. Um dies zu verhindern, wird bevorzugt eine zeitliche Regularisierung verwendet, die Sprünge zwischen GBPs bestraft. Hierzu wird der zeitliche Verlauf des optimalen Wegwertes pro GBP als beispielsweise optimale Kostenkurven (OKK) gespeichert. Derartige OKKs 40... 42 sind in Fig. 9 illustriert. Fig. 9 illustriert die OKKs 40 ... 42, die für unterschiedliche Zeiten t unterschiedliche optimale Wegwerte W aufweisen.

Die zeitliche Regularisierung beruht darauf, alle OKKs, die zu einem vorherigen Zeitpunkt nicht minimal waren, mit einem Strafterm zu belegen. In Fig. 9 ist dieser Strafterm beispielhaft durch den Doppelpfeil 45 symbolisiert. Die Kurve 44 beruht auf der OKK 41 nach der Belegung mit dem Strafterm 45 und die Kurve 43 beruht auf der OKK 42 wieder nach der Anwendung des Strafterms. Der Strafterm ist bevorzugt einfach ein additiver Wert, der vorgegeben ist und vorab beispielsweise im Rahmen einer Kalibrierung des Systems bestimmt werden kann. Auch die oben genannten Schwellwerte und Betragsschwellwerte können im Rahmen einer Kalibrierung vorab bestimmt werden. Die Positionsbestimmungseinheit ist demnach, wie oben beschrieben, bevorzugt angepasst, zu jedem Zeitpunkt von den ermittelten optimalen Wegen den optimalen Weg auszuwählen, für den ein minimaler Wegwert bestimmt worden ist, wobei, bevor der optimale Weg mit dem minimalen Wegwert ausgewählt wird, für einen optimalen Weg, für den zu früheren Zeitpunkten kein minimaler Wegwert im Vergleich zu den Wegwerten, die für die anderen optimalen Wege bestimmt worden sind, bestimmt worden ist, der Wegwert erhöht wird. Die bestimmte aktuelle Position des Katheters innerhalb der tubulären Struktur ist in Fig. 8 mit einem X markiert.

Obwohl in den obigen Ausführungsformen unterschiedliche Unähnlichkeitsmaße getrennt dargestellt worden sind, können auch Kombinationen dieser Unähnlichkeitsmaße verwendet werden. Beispielsweise können zwei oder mehrere Unähnlichkeitsmaße linear kombiniert werden, wobei dann die resultierende Linearkombination zur Positionsbestimmung verwendet werden kann.

Obwohl in den oben aufgeführten Ausführungsformen skalare Dehnungswerte entlang des medizinischen Instruments gemessen werden, um die zweite Verteilung zu bestimmen, können in anderen Ausführungsformen auch mehrere Dehnungswerte an den jeweiligen Stellen entlang des medizinischen Instruments bestimmt werden. Das heißt, die Dehnung kann auch in mindestens zwei Richtungen an der jeweiligen Stelle entlang des medizinischen Instruments bestimmt werden. Die Positionsbestimmung kann dann auf Basis dieser mehr-komponentigen Dehnungswerte und der ersten Verteilung von Krümmungswerten bestimmt werden. Zudem kann in einer weiteren Ausführungsform auch die zweite Verteilung eine Verteilung von Krümmungswerten sein, so dass die Position des medizinischen Instruments innerhalb der tubulären Struktur auf Basis von ersten und zweiten Verteilungen von Krümmungswerten bestimmt wird. Die zweite Verteilung von Krümmungswerten kann auch auf Basis von den erwähnten zwei-komponentigen oder mehrkomponentigen Dehnungswerten bestimmt werden. Wenn die Bestimmung der Position des medizinischen Instruments innerhalb der tubulären Struktur auf Basis von ersten und zweiten Verteilungen von Krümmungswerten erfolgt, sind die Krümmungswerte beider Verteilungen bevorzugt skalar.

Andere Abwandlungen der offenbarten Ausführungsformen können von Fachleuten verstanden und ausgeführt werden nach einem Studium der Zeichnung, der Beschreibung und der angefügten Ansprüche.

In den Ansprüchen schließt das Wort "umfassend" andere Elemente oder Schritte nicht aus, und der unbestimmte Artikel "ein" oder "eine" schließt eine Mehrzahl nicht aus.

Eine einzelne Einrichtung oder Vorrichtung kann die Funktionen mehrerer in den Ansprüchen angeführter Objekte erfüllen. Die bloße Tatsache, dass bestimmte Maßnahmen in voneinander verschiedenen abhängigen Ansprüchen angeführt sind, gibt nicht an, dass nicht eine Kombination dieser Maßnahmen zum Vorteil verwendet werden kann.

Verfahren, wie etwa die Bestimmung der Position des Instruments innerhalb der tubulären Struktur, die Bestimmung von Unähnlichkeitsmaßen, die Bestimmung von Wegwerten usw., die durch eine oder mehrere Einrichtungen oder Vorrichtungen durchgeführt werden, können durch eine beliebige andere Anzahl von Einrichtungen oder Vorrichtungen durchgeführt werden. Diese Verfahren und/oder die Steuerung des Bildgebungssystems gemäß dem Bildgebungsverfahren und/oder die Steuerung der Positionsbestimmungsvorrichtung gemäß dem Positionsbestimmungsverfahren können als Programmcodemittel eines Computerprogramms und/oder als dedizierte Hardware ausgeführt sein.

Ein Computerprogramm kann auf einem geeigneten Medium gespeichert/geliefert sein, wie etwa einem optischen Speichermedium oder einem Festkörpermedium, geliefert zusammen mit oder als Teil anderer Hardware, kann aber auch in anderen Formen geliefert sein, wie etwa über das Internet oder andere drahtgebundene oder drahtlose Telekommunikationssysteme.

Alle Bezugszeichen in den Ansprüchen sollten nicht als Einschränkung des Geltungsbereichs ausgelegt werden.

Die Erfindung ist in den anhängenden Ansprüchen definiert.

**Patentansprüche**

1. Positionsbestimmungsvorrichtung zum Bestimmen einer Position eines länglichen medizinischen Instruments, innerhalb einer tubulären Struktur, wobei die Positionsbestimmungsvorrichtung (5) umfasst:

   - eine erste Bereitstellungseinheit (6) zum Bereitstellen einer ersten Verteilung von Krümmungswerten an mehreren ersten Stellen entlang eines Pfades innerhalb der tubulären Struktur, wobei die erste Bereitstellungseinheit (6) angepasst ist, für mehrere Pfade innerhalb der tubulären Struktur mehrere erste Verteilungen von Krümmungswerten bereitzustellen,
   - eine zweite Bereitstellungseinheit (7) zum Bereitstellen einer zweiten Verteilung von Dehnungswerten oder von Krümmungswerten an mehreren zweiten Stellen entlang des Instruments (4), wobei die zweite Bereitstellungseinheit (7) angepasst ist, für unterschiedliche Zeitpunkte mehrere zweite Verteilungen entlang des Instruments (4) bereitzustellen, wobei die mehreren zweiten Verteilungen mehreren Positionen des Instruments (4) relativ zu einem Pfad der tubulären Struktur entsprechen,
   - eine Positionsbestimmungseinheit (8), die angepasst ist,

     - für jeden Pfad und für jeden Zeitpunkt jeweils eine Verteilung von Unähnlichkeitswerten für unterschiedliche Kandidatenpositionen des Instruments (4) relativ zu dem jeweiligen Pfad zu bestimmen, wobei ein Unähnlichkeitsmaß auf die entsprechende erste Verteilung und die entsprechende zweite Verteilung für verschiedene mögliche Kandidatenpositionen des Instruments (4) relativ zu dem Pfad angewendet wird, wobei das Unähnlichkeitsmaß angepasst ist, für jede Kandidatenposition des Instruments (4) relativ zu dem jeweiligen Pfad einen Unähnlichkeitswert zu liefern, der indikativ ist für eine Unähnlichkeit der beiden Verteilungen in der jeweiligen Kandidatenposition, wobei die für die unterschiedlichen Zeitpunkte und die unterschiedlichen Kandidatenpositionen bestimmten Unähnlichkeitswerte als eine Karte auffassbar sind, wobei für unterschiedliche Orte, die durch die jeweiligen Zeitpunkte und Kandidatenpositionen festgelegt sind, in der Karte jeweils ein Unähnlichkeitswert eingetragen ist,
     - ein Wegmaß bereitzustellen, das für einen Weg durch die jeweilige Karte, der bei einer Kandidatenposition für einen spätesten Zeitpunkt endet und bei einer Kandidatenposition für einen früheren Zeitpunkt beginnt, einen Wegwert liefert, der von den Unähnlichkeitswerten entlang des jeweiligen Weges abhängt,
     - für jeden Pfad einen Weg durch die jeweilige Karte mittels des Wegma-ßes zu bestimmen, für den ein minimaler Wegwert bestimmt wird, so dass für unterschiedliche Pfade und damit für unterschiedliche Karten jeweils ein optimaler Weg ermittelt wird, und
     - die Positionen des Instruments (4) relativ zu einem der Pfade auf Basis der für die unterschiedlichen Pfade ermittelten optimalen Wege unter Berücksichtigung der für diese optimalen Wege bestimmten Wegwerte zu bestimmen.

2. Positionsbestimmungsvorrichtung nach Anspruch 1, **dadurch gekennzeichnet, dass** die erste Bereitstellungseinheit (6) angepasst ist, eine skalare Verteilung von Krümmungswerten entlang eines Pfades innerhalb der tubulären Struktur als die erste Verteilung bereitzustellen.

3. Positionsbestimmungsvorrichtung nach einem der vorhergehenden Ansprüche, **dadurch gekennzeichnet, dass** die zweite Bereitstellungseinheit (7) angepasst ist, die zweite Verteilung auf Basis von optischen Signalen optischer Dehnungssensoren zu bestimmen, die entlang des Instruments (4) angeordnet sind.

4. Positionsbestimmungsvorrichtung nach einem der vorhergehenden Ansprüche, **dadurch gekennzeichnet, dass** die Positionsbestimmungseinheit (8) angepasst ist, ein Wegmaß bereitzustellen, das eine Komponente aufweist, die mit zunehmender Summe der Unähnlichkeitswerte entlang des jeweiligen Weges größer wird.

5. Positionsbestimmungsvorrichtung nach einem der vorgehenden Ansprüche, **dadurch gekennzeichnet, dass** die Positionsbestimmungseinheit (8) angepasst ist,

   - für unterschiedliche Zeitpunkte optimale Wege zu ermitteln, so dass für unterschiedliche Zeitpunkte und unterschiedliche Pfade jeweils ein optimaler Weg mit einem entsprechenden Wegwert bestimmt wird, wobei, um für einen Pfad und einen bestimmten Zeitpunkt einen optimalen Weg mit einem entsprechenden Wegwert zu bestimmen, die für den bestimmten Zeitpunkt und für frühere Zeitpunkte und für die Kandidatenpositionen ermittelten Unähnlichkeitswerte, die als eine Karte für den jeweiligen Pfad und den bestimmten Zeitpunkt auffassbar sind, verwendet werden,

- zu jedem Zeitpunkt von den ermittelten optimalen Wegen den optimalen Weg auszuwählen, für den ein minimaler Wegwert bestimmt worden ist, wobei, bevor der optimale Weg mit dem minimalen Wegwert ausgewählt wird, für einen optimalen Weg, für den zu früheren Zeitpunkten kein minimaler Wegwert im Vergleich zu den Wegwerten, die für die anderen optimalen Wege bestimmt worden sind, bestimmt worden ist, der Wegwert erhöht wird,

- als die Positionen des Instruments (4) relativ zu einem der Pfade die Positionen zu bestimmen, die durch den ausgewählten optimalen Weg definiert sind.

6. Positionsbestimmungsvorrichtung nach einem der vorhergehenden Ansprüche, **dadurch gekennzeichnet, dass** die Positionsbestimmungseinheit (8) angepasst ist, das Unähnlichkeitsmaß anzuwenden, indem

- eine räumliche erste Gradientenverteilung der ersten Verteilung bestimmt wird,
- eine räumliche zweite Gradientenverteilung der zweiten Verteilung bestimmt wird,
- für jeden Ort entlang des Instruments (4) der jeweilige Gradient der zweiten Verteilung mit dem jeweiligen Krümmungsgradienten der ersten Verteilung verglichen wird, wobei die jeweilige Kandidatenposition definiert, welcher jeweilige Gradient der zweiten Verteilung mit welchem jeweiligen Krümmungsgradienten der ersten Verteilung an dem jeweiligen Ort verglichen wird, wobei für den jeweiligen Ort und für die jeweilige Kandidatenposition ein Unter-Unähnlichkeitsmaß verwendet wird, das abhängig ist von a) den Richtungen der Gradienten relativ zueinander und/oder b) dem Betrag des Gradienten der ersten Verteilung und dem Betrag des Gradienten der zweiten Verteilung, wobei durch die Anwendung des Unter-Unähnlichkeitsma-ßes für jeden Ort ein Unter-Unähnlichkeitswert bestimmt wird,
- die Unter-Unähnlichkeitswerte, die für eine Kandidatenposition bestimmt worden sind, summiert werden, um für die jeweilige Kandidatenposition einen jeweiligen Unähnlichkeitswert zu bestimmen.

7. Positionsbestimmungsvorrichtung nach einem der vorhergehenden Ansprüche, **dadurch gekennzeichnet, dass** die Positionsbestimmungseinheit (8) angepasst ist, das Unähnlichkeitsmaß anzuwenden, indem

- lokale Maxima der ersten Verteilung bestimmt werden,
- lokale Maxima der zweiten Verteilung bestimmt werden,
- jedem lokalen Maximum der ersten Verteilung ein lokales Maximum der zweiten Verteilung zugeordnet wird, so dass die Summe aller räumlichen Abstände zwischen zugeordneten lokalen Maxima minimal ist, wobei jedes lokale Maximum der zweiten Verteilung nur einem lokalen Maximum der ersten Verteilung zugeordnet wird,
- die räumlichen Abstände zwischen den zugeordneten lokalen Maxima der ersten Verteilung und den zugeordneten lokalen Maxima der zweiten Verteilung addiert werden.

8. Positionsbestimmungsvorrichtung nach einem der vorhergehenden Ansprüche, **dadurch gekennzeichnet, dass** die Positionsbestimmungseinheit (8) angepasst ist, das Unähnlichkeitsmaß anzuwenden, indem

- für jeden Ort entlang des Instruments (4) der jeweilige Wert der zweiten Verteilung mit dem jeweiligen Wert der ersten Verteilung verglichen wird, wobei die jeweilige Kandidatenposition definiert, welcher jeweilige Wert der zweiten Verteilung mit welchem jeweiligen Wert der ersten Verteilung an dem jeweiligen Ort verglichen wird, wobei für den jeweiligen Ort und für die jeweilige Kandidatenposition ein Unter-Unähnlichkeitsmaß verwendet wird, das Null ist, wenn a) der jeweilige Wert der ersten Verteilung oberhalb eines vorgegebenen ersten Schwellwerts liegt oder b) der jeweilige Wert der ersten Verteilung unterhalb des ersten Schwellwerts liegt und der jeweilige Wert der zweiten Verteilung unterhalb eines vorgegebenen zweiten Schwellwerts liegt, und das ansonsten einen positiven Wert aufweist,
- die Unter-Unähnlichkeitswerte, die für eine Kandidatenposition bestimmt worden sind, zu summieren, um für die jeweilige Kandidatenposition einen jeweiligen Unähnlichkeitswert zu bestimmen.

9. Positionsbestimmungsvorrichtung nach einem der vorhergehenden Ansprüche, **dadurch gekennzeichnet, dass** die Positionsbestimmungseinheit (8) angepasst ist, das Unähnlichkeitsmaß anzuwenden, indem eine Kreuzkorrelation, insbesondere eine normalisierte Kreuzkorrelation, auf die erste Verteilung und die zweite Verteilung für die jeweilige Kandidatenposition angewendet wird.

10. Bildgebungssystem, umfassend:

- eine Positionsbestimmungsvorrichtung (5) zum Bestimmen einer Position eines länglichen medizinischen Instruments (4) innerhalb einer tubulären Struktur nach Anspruch 1,
- eine bildgebende Einheit (10) zum Bereitstellen eines Bilds der tubulären Struktur,
- eine Visualisierungserzeugungseinheit (11) zum Erzeugen einer Visualisierung der tubulären Struktur auf Grundlage des bereitgestellten Bilds und der bestimmten Position.

**11.** Positionsbestimmungsverfahren zum Bestimmen einer Position eines länglichen medizinischen Instruments (4) innerhalb einer tubulären Struktur, wobei das Verfahren umfasst:

- Bereitstellen einer ersten Verteilung von Krümmungswerten an mehreren ersten Stellen entlang eines Pfades innerhalb der tubulären Struktur durch eine erste Bereitstellungseinheit (6), wobei für mehrere Pfade innerhalb der tubulären Struktur mehrere erste Verteilungen von Krümmungswerten bereitgestellt werden,
- Bereitstellen einer zweiten Verteilung von Dehnungswerten oder von Krümmungswerten an mehreren zweiten Stellen entlang des Instruments (4) durch eine zweite Bereitstellungseinheit (7), wobei für unterschiedliche Zeitpunkte mehrere zweite Verteilungen entlang des Instruments bereitgestellt werden, wobei die mehreren zweiten Verteilungen mehreren Positionen des Instruments (4) relativ zu einem Pfad der tubulären Struktur entsprechen,
- Bestimmen, für jeden Pfad und für jeden Zeitpunkt, von jeweils einer Verteilung von Unähnlichkeitswerten für unterschiedliche Kandidatenpositionen des Instruments (4) relativ zu dem jeweiligen Pfad durch eine Positionsbestimmungseinheit (8), wobei ein Unähnlichkeitsmaß auf die entsprechende erste Verteilung und die entsprechende zweite Verteilung für verschiedene mögliche Kandidatenpositionen des Instruments (4) relativ zu dem Pfad angewendet wird, wobei das Unähnlichkeitsmaß angepasst ist, für jede Kandidatenposition des Instruments (4) relativ zu dem jeweiligen Pfad einen Unähnlichkeitswert zu liefern, der indikativ ist für eine Unähnlichkeit der beiden Verteilungen in der jeweiligen Kandidatenposition, wobei die für die unterschiedlichen Zeitpunkte und die unterschiedlichen Kandidatenpositionen bestimmten Unähnlichkeitswerte als eine Karte auffassbar sind, wobei für unterschiedliche Orte, die durch die jeweiligen Zeitpunkte und Kandidatenpositionen festgelegt sind, in der Karte jeweils ein Unähnlichkeitswert eingetragen ist,
- Bereitstellen eines Wegmaßes, das für einen Weg durch die jeweilige Karte, der bei einer Kandidatenposition für einen spätesten Zeitpunkt endet und bei einer Kandidatenposition für einen früheren Zeitpunkt beginnt, einen Wegwert liefert, der von den Unähnlichkeitswerten entlang des jeweiligen Weges abhängt, durch die Positionsbestimmungseinheit (8),
- Bestimmen eines Weges für jeden Pfad durch die jeweilige Karte mittels des Wegmaßes, für den ein minimaler Wegwert bestimmt wird, so dass für unterschiedliche Pfade und damit für unterschiedliche Karten jeweils ein optimaler Weg ermittelt wird, durch die Positionsbestimmungseinheit (8), und
- Bestimmen der Positionen des Instruments (4) relativ zu einem der Pfade auf Basis der für die unterschiedlichen Pfade ermittelten optimalen Wege unter Berücksichtigung der für diese optimalen Wege bestimmten Wegwerte durch die Positionsbestimmungseinheit (8).

**12.** Bildgebungsverfahren, umfassend:

- Bereitstellen eines Bilds einer tubulären Struktur durch eine bildgebende Einheit (10),
- Bestimmen einer Position eines länglichen medizinischen Instruments (4) innerhalb der tubulären Struktur gemäß dem Positionbestimmungsverfahren nach Anspruch 11 durch eine Positionsbestimmungsvorrichtung nach Anspruch 1, und
- Erzeugen einer Visualisierung der tubulären Struktur auf Grundlage des bereitgestellten Bilds und der bestimmten Position durch eine Visualisierungserzeugungseinheit.

**13.** Computerprogramm zum Bestimmen einer Position eines länglichen medizinischen Instruments, wobei das Computerprogramm Programmcodemittel aufweist, die angepasst sind, die Positionsbestimmungsvorrichtung nach Anspruch 1 dazu zu veranlassen, das Positionsbestimmungsverfahren zum Bestimmen einer Position eines länglichen medizinischen Instruments (4) innerhalb einer tubulären Struktur gemäß Anspruch 11 durchzuführen, wenn es auf der Positionsbestimmungsvorrichtung (1) ausgeführt wird.

**14.** Computerprogramm nach Anspruch 13, **dadurch gekennzeichnet, dass** die Programmcodemittel des Weiteren ausgebildet sind, ein Bildgebungssystems nach Anspruch 10, das die Positionsbestimmungsvorrichtung umfasst, zu veranlassen, folgende Schritte auszuführen, wenn das Computerprogramm auf dem Bildgebungssystem ausgeführt wird:

- Bereitstellen eines Bilds der tubulären Struktur, und
- Erzeugen einer Visualisierung der tubulären Struktur auf Grundlage des bereitgestellten Bilds und der bestimmten Position.

**Claims**

1. A position determining device for determining the position of an elongate medical instrument within a tubular structure, said position determining device (5) comprising:

   - a first providing unit (6) for providing a first distribution of curvature values at a plurality of first points along a path within the tubular structure, wherein the first providing unit (6) is adapted to provide a plurality of first distributions of curvature values for a plurality of paths within the tubular structure,
   - a second providing unit (7) for providing a second distribution of strain values or curvature values at a plurality of second points along the instrument (4), wherein the second providing unit (7) is adapted to provide a plurality of second distributions along the instrument (4) for different points in time, wherein the plurality of second distributions correspond to a plurality of positions of the instrument (4) relative to a path within the tubular structure,
   - a position determining unit (8), adapted to

     - to determine for each path and for each point in time a distribution of dissimilarity scores for different candidate positions of the instrument (4) relative to the respective path, wherein a dissimilarity measure to the corresponding first distribution and the corresponding second distribution for different possible candidate positions of the instrument (4) relative to the path is applied, wherein the dissimilarity measure is adapted to return a dissimilarity score indicative of a dissimilarity of the two distributions in the respective candidate position for each candidate position of the instrument (4) relative to the path, wherein dissimilarity scores determined for the different points in time and the different candidate positions can be interpreted as a map, wherein a respective dissimilarity score is entered in the map for different locations specified by the respective points in time and candidate positions,
     - to provide a route measure which returns a route score for a route through the respective map that ends at a candidate position for a latest point in time and begins at a candidate position for an earlier point in time, said route score depending on the dissimilarity scores along the respective route,
     - to determine for each path a route through the respective map by means of the route measure, for which a minimum route score is determined such that a respective optimal route is calculated for different paths and thus for different maps, and
     - to determine the positions of the instrument (4) relative to one of the paths on the basis of optimal routes calculated for the different paths by taking into account the route scores determined for said optimal routes.

2. The position determining device according to claim 1, **characterised in that** the first providing unit (6) is adapted to provide a scalar distribution of curvature values along a path within the tubular structure as the first distribution.

3. The position determining device according to any one of the preceding claims, **characterised in that** the second providing unit (7) is adapted to determine the second distribution on the basis of optical signals from optical strain sensors arranged along the instrument (4).

4. The position determining device according to any one of the preceding claims, **characterised in that** the position determining unit (8) is adapted to provide a route measure which includes a component that becomes greater as the sum of the dissimilarity scores increases along the respective route.

5. The position determining device according to any one of the preceding claims, **characterised in that** the position determining unit (8) is adapted

   - to calculate optimal routes for different points in time such that a respective optimal route with a corresponding route score is determined for different points in time and different paths, wherein, in order to determine an optimal route with a corresponding route score for a path and a particular point in time, use is made of the dissimilarity scores calculated for the particular point in time and for earlier points in time and for the candidate positions, which can be interpreted as a map for the respective route and the particular point in time,
   - to select at each point in time, from the calculated optimal routes, the optimal route for which a minimum route score has been determined, wherein the route score is increased, before the optimal route with the minimum route score is selected, for an optimal route for which no minimum route score was determined at earlier points in time compared to the route scores determined for the other optimal routes,

- to specify the positions defined by the selected optimal route as the positions of the instrument (4) relative to one of the paths.

6. The position determining device according to any one of the preceding claims, **characterised in that** the position determining unit (8) is adapted to apply the dissimilarity measure by

- determining a spatial first gradient distribution of the first distribution,
- determining a spatial second gradient distribution of the second distribution,
- comparing, for each location along the instrument (4), the respective gradient of the second distribution with the respective curvature gradients of the first distribution, wherein the respective candidate position defines which respective gradient of the second distribution is compared with which respective curvature gradient of the first distribution at the respective location, wherein, for the respective location and for the respective candidate position, a sub-dissimilarity measure is applied which is dependent a) on the directions of the gradients relative to each other and/or b) on the amount of the gradient of the first distribution and the amount of the gradient of the second distribution, wherein a sub-dissimilarity score is determined for each location by applying the sub-dissimilarity measure,
- summing the sub-dissimilarity scores determined for a candidate position, in order to determine a respective dissimilarity score for the respective candidate position.

7. The position determining device according to any one of the preceding claims, **characterised in that** the position determining unit (8) is adapted to apply the dissimilarity measure by

- determining local maxima of the first distribution,
- determining local maxima of the second distribution,
- assigning a local maximum of the second distribution to each local maximum of the first distribution, so that the sum of all the spatial distances between assigned local maxima is minimal, wherein each local maximum of the second distribution is assigned to only one local maximum of the first distribution,
- adding the spatial distances between the assigned local maxima of the first distribution and the assigned local maxima of the second distribution.

8. The position determining device according to any one of the preceding claims, **characterised in that** the position determining unit (8) is adapted to apply the dissimilarity measure by

- comparing the respective value of the second distribution with the respective value of the first distribution for each location along the instrument (4), wherein the respective candidate position defines which respective value of the second distribution is compared with which respective value of the first distribution at the respective location, wherein, for the respective location and for the respective candidate position, a sub-dissimilarity measure is used which is zero if a) the respective value of the first distribution is above a predefined first threshold or b) the respective value of the first distribution is below the first threshold and the respective value of the second distribution is below a predefined second threshold, and which otherwise has a positive value,
- summing the sub-dissimilarity scores determined for a candidate position, in order to determine a respective dissimilarity score for the respective candidate position.

9. The position determining device according to any one of the preceding claims, **characterised in that** the position determining unit (8) is adapted to apply the dissimilarity measure by applying a cross-correlation, in particular a normalised cross-correlation, to the first distribution and the second distribution for the respective candidate position.

10. An imaging system, comprising:

- a position determining device (5) for determining the position of an elongate medical instrument (4) within a tubular structure in accordance with claim 1,
- an imaging unit (10) for providing an image of the tubular structure,
- a visualisation generating unit (11) for generating a visualisation of the tubular structure on the basis of the image provided and the position determined.

11. A position determining method for determining the position of an elongate medical instrument (4) within a tubular structure, said method comprising the steps of:

- providing a first distribution of curvature values at a plurality of first points along a path within the tubular structure by a first providing unit (6), wherein a plurality of first distributions of curvature values for a plurality of paths within the tubular structure is provided,
- providing a second distribution of strain values or curvature values at a plurality of second points along the instrument (4) by a second providing unit (7), wherein a plurality of second distributions along the instrument (4) for different points in time is provided, wherein the plurality of second distributions correspond to a plurality of positions of the instrument (4) relative to a path within the tubular structure,
- determining for each path and for each point in time a distribution of dissimilarity scores for different candidate positions of the instrument (4) relative to the respective path by a position determining unit (8), wherein a dissimilarity measure to the corresponding first distribution and the corresponding second distribution for different possible candidate positions of the instrument (4) relative to the path is applied, wherein the dissimilarity measure is adapted to return a dissimilarity score indicative of a dissimilarity of the two distributions in the respective candidate position for each candidate position of the instrument (4) relative to the path, wherein dissimilarity scores determined for the different points in time and the different candidate positions can be interpreted as a map, wherein a respective dissimilarity score is entered in the map for different locations specified by the respective points in time and candidate positions,
- providing a route measure which returns a route score for a route through the respective map that ends at a candidate position for a latest point in time and begins at a candidate position for an earlier point in time, said route score depending on the dissimilarity scores along the respective route by the position determining unit (8),
- determining for each path a route through the respective map by means of the route measure, for which a minimum route score is determined such that a respective optimal route is calculated for different paths and thus for different maps by the position determining unit (8), and
- determining the positions of the instrument (4) relative to one of the paths on the basis of optimal routes calculated for the different paths by taking into account the route scores determined for said optimal routes by the position determining unit (8).

12. An imaging method, comprising the steps of:

- providing an image of a tubular structure by an imaging unit (10),
- determining the position of an elongate medical instrument (4) within the tubular structure according to the position determining method according to claim 11 by a position determining device according to claim 1, and
- a visualisation generating unit generating a visualisation of the tubular structure on the basis of the image provided and the position determined.

13. A computer program for determining the position of an elongate medical instrument, wherein the computer program includes program code means adapted to cause the position determining device according to claim 1 to carry out the position determining method for determining the position of an elongate medical instrument (4) within a tubular structure according to claim 11 when it is executed on the position determining device (1).

14. The computer program according to claim 13, **characterized in that** the program code means are further adapted to cause the imaging system according to claim 10 comprising the position determining device to carry out, when the computer program is executed on the imaging system, following steps:

- providing an image of the tubular structure
- generating a visualisation of the tubular structure on the basis of the image provided and the position determined.

## Revendications

1. Dispositif de détermination de position destiné à déterminer une position d'un instrument médical allongé à l'intérieur d'une structure tubulaire, dans lequel le dispositif de détermination de position (5) comprend :

- une première unité de fourniture (6) destinée à fournir une première distribution de valeurs de courbure sur plusieurs premiers emplacements le long d'un trajet à l'intérieur de la structure tubulaire, dans lequel la première unité de fourniture (6) est adaptée pour fournir plusieurs premières distributions de valeurs de courbure pour plusieurs trajets à l'intérieur de la structure tubulaire,
- une deuxième unité de fourniture (7) destinée à fournir une deuxième distribution de valeurs d'allongement ou de valeurs de courbures sur plusieurs deuxièmes emplacements le long de l'instrument (4), dans lequel la deuxième unité de fourniture (7) est adaptée pour fournir plusieurs deuxièmes distributions le long de l'instrument (4) pour différents moments, dans lequel les plusieurs deuxièmes distributions correspondent à plusieurs positions de l'instrument (4) par rapport à un trajet de la structure tubulaire,
- une unité de détermination de position (8), qui est adaptée

-- pour déterminer, pour chaque trajet et pour chaque moment, respectivement une distribution de valeurs de dissimilarité pour différentes positions candidates de l'instrument (4) par rapport au trajet respectif, dans lequel une mesure de dissimilarité est appliquée sur la première distribution correspondante et la deuxième distribution correspondante pour différentes positions candidates possibles de l'instrument (4) par rapport au trajet, dans lequel la mesure de dissimilarité est adaptée pour fournir une valeur de dissimilarité pour chaque position candidate de l'instrument (4) par rapport au trajet respectif, laquelle indique une dissimilarité des deux distributions dans la position candidate respective, dans lequel les valeurs de dissimilarité déterminées pour les différents moments et les différentes positions candidates peuvent être considérées comme une carte, dans lequel respectivement une valeur de dissimilarité est enregistrée sur la carte pour différents lieux, qui sont fixés par les moments et les positions candidates respectivement,
-- pour fournir une mesure de déplacement, qui offre une valeur de déplacement, laquelle dépend des valeurs de dissimilarité le long du déplacement respectif, pour un déplacement à travers la carte respective, qui se termine à une position candidate pour un moment au plus tard et commence à une position candidate pour un moment antérieur
-- pour déterminer pour chaque trajet un déplacement à travers la carte respective au moyen de la mesure de déplacement, pour laquelle une valeur de déplacement minimale est déterminée de telle sorte que respectivement un déplacement optimal est calculé pour différents trajets et ainsi pour différentes cartes, et
-- pour déterminer les positions de l'instrument (4) par rapport à un des trajets sur la base des déplacements optimaux calculés pour les différents trajets en tenant compte des valeurs de déplacement déterminées pour lesdits déplacements optimaux.

2. Dispositif de détermination de position selon la revendication 1, **caractérisé en ce que** la première unité de fourniture (6) est adaptée pour fournir, en tant que la première distribution, une distribution scalaire de valeurs de courbure le long d'un trajet à l'intérieur de la structure tubulaire.

3. Dispositif de détermination de position selon l'une quelconque des revendications précédentes, **caractérisé en ce que** la deuxième unité de fourniture (7) est adaptée pour déterminer la deuxième distribution sur la base de signaux optiques de capteurs d'allongement optiques, qui sont disposés le long de l'instrument (4).

4. Dispositif de détermination de position selon l'une quelconque des revendications précédentes, **caractérisé en ce que** l'unité de détermination de position (8) est adaptée pour fournir une mesure de déplacement, qui présente une composante qui augmente au fur et à mesure que la somme des valeurs de dissimilarité augmente le long du déplacement respectif.

5. Dispositif de détermination de position selon l'une quelconque des revendications précédentes, **caractérisé en ce que** l'unité de détermination de position (8) est adaptée

- pour calculer des déplacements optimaux pour différents moments de telle sorte que respectivement un déplacement optimal avec une valeur de déplacement correspondante est déterminé pour différents moments et différents trajets, dans lequel, pour déterminer, pour un trajet et un moment déterminé, un déplacement optimal avec une valeur de déplacement correspondante, les valeurs de dissimilarité calculées pour le moment déterminé et des moments antérieurs et pour les positions candidates, qui sont considérées comme une carte pour le trajet respectif et le moment respectif, sont utilisées,
- pour sélectionner, pour chaque moment, parmi les déplacements optimaux calculés, le déplacement optimal, pour lequel une valeur de déplacement minimale a été déterminée, dans lequel, avant que le déplacement optimal avec la valeur de déplacement minimale ne soit sélectionné, aucune valeur de déplacement minimale n'a été déterminée en comparaison avec les valeurs de déplacement, qui ont été déterminées pour les autres déplacements optimaux, pour un déplacement optimal, pour les moments trop précoces, la valeur de déplacement est augmentée,
- pour déterminer les positions, qui sont définies par le déplacement optimal sélectionné, en tant que les positions de l'instrument (4) par rapport à un des trajets.

6. Dispositif de détermination de position selon l'une quelconque des revendications précédentes, **caractérisé en ce que** l'unité de détermination de position (8) est adaptée pour appliquer la mesure de dissimilarité, **en ce que**

- une première distribution de gradient spatiale de la première distribution est déterminée,
- une deuxième distribution de gradient spatiale de la deuxième distribution est déterminée,
- le gradient respectif de la deuxième distribution est comparé au gradient de courbure respectif de la première distribution pour chaque lieu le long de l'instrument (4), dans lequel la position candidate respective définit le gradient respectif de la deuxième distribution et le gradient de courbure respectif de la première distribution sur le lieu respectif, auquel le premier est comparé, dans lequel une mesure inférieure de dissimilarité est utilisée pour le lieu respectif et pour la position candidate respective, qui dépend a) des directions des gradients les uns par rapport aux autres et/ou b) de la valeur du gradient de la première distribution et de la valeur du gradient de la deuxième distribution, dans lequel une valeur inférieure de dissimilarité est déterminée par l'application de la valeur inférieure de dissimilarité pour chaque lieu,
- les valeurs inférieures de dissimilarité, qui ont été déterminées pour une position candidate, sont additionnées pour déterminer, pour la position candidate respective, une valeur de dissimilarité respective.

7. Dispositif de détermination de position selon l'une quelconque des revendications précédentes, **caractérisé en ce que** l'unité de détermination de position (8) est adaptée pour appliquer la mesure de dissimilarité, **en ce que**

- des valeurs maximales locales de la première distribution sont déterminées,
- des valeurs maximales locales de la deuxième distribution sont déterminées,
- une valeur maximale locale de la deuxième distribution est associée à chaque valeur maximale locale de la première distribution de telle sorte que la somme de toutes les distances spatiales entre des valeurs maximales locales associées est minimale, dans lequel chaque valeur maximale locale de la deuxième distribution est associée seulement à une valeur maximale locale de la première distribution,
- les distances spatiales entre les valeurs maximales locales associées de la première distribution et les valeurs maximales locales associées de la deuxième distribution sont additionnées.

8. Dispositif de détermination de position selon l'une quelconque des revendications précédentes, **caractérisé en ce que** l'unité de détermination de position (8) est adaptée pour appliquer la mesure de dissimilarité, **en ce que**

- la valeur respective de la deuxième distribution est comparée à la valeur respective de la première distribution pour chaque lieu le long de l'instrument (4), dans lequel la position candidate respective définit la valeur respective de la deuxième distribution et la valeur respective de la première distribution sur le lieu respectif, à laquelle la première est comparée, dans lequel est utilisée, pour le lieu respectif et pour la position candidate respective, une valeur inférieure de dissimilarité, laquelle est nulle lorsque a) la valeur respective de la première distribution est supérieure à une première valeur de seuil spécifiée ou b) la valeur respective de la première distribution est inférieure à la première valeur de seuil, et la valeur respective de la deuxième distribution est inférieure à une deuxième valeur de seuil spécifiée, et qui présente le cas échéant une valeur positive,
- pour additionner les valeurs inférieures de dissimilarité, qui ont été déterminées pour une position candidate pour déterminer, pour la position candidate respective, une valeur de dissimilarité respective.

9. Dispositif de détermination de position selon l'une quelconque des revendications précédentes, **caractérisé en ce que** l'unité de détermination de position (8) est adaptée pour appliquer la mesure de dissimilarité **en ce qu'**une corrélation croisée, en particulier une corrélation croisée normalisée, est appliquée sur la première distribution et la deuxième distribution pour la position candidate respective.

10. Système d'imagerie comprenant :

- un dispositif de détermination de position (5) destiné à déterminer une position d'un instrument médical allongé (4) à l'intérieur d'une structure tubulaire selon la revendication 1,
- une unité d'imagerie (10) destinée à fournir une image de la structure tubulaire,
- une unité de production de visualisation (11) destinée à générer une visualisation de la structure tubulaire sur la base de l'image fournie et de la position déterminée.

11. Procédé de détermination de position destiné à déterminer une position d'un instrument médical allongé (4) à l'intérieur d'une structure tubulaire, dans lequel le procédé comprend :

- la fourniture d'une première distribution de valeurs de courbure sur plusieurs premiers emplacements le long d'un trajet à l'intérieur de la structure tubulaire par une première unité de fourniture (6), dans lequel plusieurs premières distributions de valeurs de courbure sont fournies pour plusieurs trajets à l'intérieur de la structure tubulaire,

- la fourniture d'une deuxième distribution de valeurs d'allongement ou de valeurs de courbures sur plusieurs deuxièmes emplacements le long de l'instrument (4) par une deuxième unité de fourniture (7), dans lequel les plusieurs deuxièmes distributions le long de l'instrument sont fournies pour différents moments, dans lequel les plusieurs deuxièmes distributions correspondent à plusieurs positions de l'instrument (4) par rapport à un trajet de la structure tubulaire,

- la détermination, pour chaque trajet et pour chaque moment, respectivement d'une distribution de valeurs de dissimilarité pour différentes positions candidates de l'instrument (4) par rapport au trajet respectif par une unité de détermination de position (8), dans lequel une mesure de dissimilarité est appliquée sur la première distribution correspondante et la deuxième distribution correspondante pour différentes positions candidates possibles de l'instrument (4) par rapport au trajet, dans lequel la mesure de dissimilarité est adaptée pour fournir une valeur de dissimilarité pour chaque position candidate de l'instrument (4) par rapport au trajet respectif, laquelle indique une dissimilarité des deux distributions dans la position candidate respective, dans lequel les valeurs de dissimilarité déterminées pour les différents moments et les différentes positions candidates peuvent être considérées comme une carte, dans lequel respectivement une valeur de dissimilarité est enregistrée sur la carte pour différents lieux, qui sont fixés par les moments et les positions candidates respectivement,

- la fourniture d'une mesure de déplacement, qui offre une valeur de déplacement pour un déplacement à travers la carte respective, qui se termine à une position candidate pour un moment au plus tard et commence à une position candidate pour un moment antérieur, laquelle dépend des valeurs de dissimilarité le long du déplacement respectif, par l'unité de détermination de position (8),

- la détermination d'un déplacement pour chaque trajet à travers la carte respective au moyen de la mesure de déplacement, pour laquelle une valeur de déplacement minimale est déterminée de telle sorte que respectivement un déplacement optimal est calculé pour différents trajets et ainsi pour différentes cartes, par l'unité de détermination de position (8), et

- la détermination des positions de l'instrument (4) par rapport à un des trajets sur la base des déplacements optimaux calculés pour les différents trajets en tenant compte des valeurs de déplacement déterminées pour lesdits déplacements optimaux par l'unité de détermination de position (8).

12. Procédé d'imagerie comprenant :

- la fourniture d'une image d'une structure tubulaire par une unité d'imagerie (10),
- la détermination d'une position d'un instrument médical allongé (4) à l'intérieur de la structure tubulaire selon le procédé de détermination de position selon la revendication 11 par un dispositif de détermination de position selon la revendication 1, et
- la génération d'une visualisation de la structure tubulaire sur la base de l'image fournie et de la position déterminée par une unité de génération de visualisation.

13. Programme d'ordinateur destiné à déterminer une position d'un instrument médical allongé, dans lequel le programme d'ordinateur présente des moyens de code de programme, qui sont adaptés pour amener le dispositif de détermination de position selon la revendication 1 à mettre en oeuvre le procédé de détermination de position pour déterminer une position d'un instrument médical allongé (4) à l'intérieur d'une structure tubulaire selon la revendication 11 lorsqu'il est exécuté sur le dispositif de détermination de position (1).

14. Programme d'ordinateur selon la revendication 13, **caractérisé en ce que** les moyens de code de programme sont réalisés par ailleurs pour amener un système d'imagerie selon la revendication 10, qui comprend le dispositif de détermination de position, à exécuter des étapes suivantes lorsque le programme d'ordinateur est exécuté sur le système d'imagerie :

- la fourniture d'une image de la structure tubulaire, et
- la génération d'une visualisation de la structure tubulaire sur la base de l'image fournie et de la position déterminée.

**FIG. 1**

FIG. 2

FIG. 3

FIG. 4

FIG. 5

p

FIG. 6

FIG. 7

FIG. 8

FIG. 9

## IN DER BESCHREIBUNG AUFGEFÜHRTE DOKUMENTE

### In der Beschreibung aufgeführte Patentdokumente

- US 2015254526 A1
- US 20160302869 A1
- US 20140336501 A1